(19) Europäisches Patentamt
European Patent Office
Office européen des brevets

(11) EP 1 838 671 B1

(12) **EUROPEAN PATENT SPECIFICATION**

(45) Date of publication and mention of the grant of the patent:
**04.06.2008 Bulletin 2008/23**

(21) Application number: **05826014.2**

(22) Date of filing: **05.12.2005**

(51) Int Cl.:
***C07D 209/88*** *(2006.01)*

(86) International application number:
**PCT/IB2005/054039**

(87) International publication number:
**WO 2006/061759 (15.06.2006 Gazette 2006/24)**

(54) **9,9'- AND 2,2'-SUBSTITUTED 3,3'-BICARBAZOLYL DERIVATIVES FOR USE IN SEMICONDUCTING MATERIALS AS A HOST MATRIX FOR PHOSPHORESCENT EMITTERS**

9,9'- UND 2,2'-SUBSTITUIERTE 3,3'-BICARBAZOLYL-DERIVATE ZUR VERWENDUNG IN HALBLEITENDEN MATERIALIEN ALS GASTMATRIX FÜR PHOSPHORESZIERENDE EMITTER

DÉRIVÉS 3,3'-BICARBAZOLYL SUBSTITUÉS 9,9' ET 2,2' POUR L'UTILISATION DANS DES MATERIELS SEMICONDUCTEURS COMME MATRIX HÔTE POUR EMMITEURS PHOSPHORESCENTS

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.12.2004 EP 04106381**

(43) Date of publication of application:
**03.10.2007 Bulletin 2007/40**

(73) Proprietor: **Koninklijke Philips Electronics N.V.**
**5621 BA Eindhoven (NL)**

(72) Inventors:
- **BRUNNER, Klemens**
  **NL-5656 AA Eindhoven (NL)**
- **VAN DIJKEN, Addy**
  **NL-5656 AA Eindhoven (NL)**
- **HOFSTRAAT, Johannes, W.**
  **NL-5656 AA Eindhoven (NL)**
- **BOERNER, Herbert, F.**
  **NL-5656 AA Eindhoven (NL)**
- **LANGEVELD-VOSS, Bea, M., W.**
  **NL-5656 AA Eindhoven (NL)**
- **KIGGEN, Nicole, M., T.**
  **NL-5656 AA Eindhoven (NL)**
- **BASTIAANSEN, Jolanda, J., A., M.**
  **NL-5656 AA Eindhoven (NL)**
- **SCHOO, Hermannus, F., M.**
  **NL-5656 AA Eindhoven (NL)**

(74) Representative: **Rolfes, Johannes Gerardus Albertus**
**Philips**
**Intellectual Property & Standards**
**P.O. Box 220**
**5600 AE Eindhoven (NL)**

(56) References cited:
**WO-A-20/04055129** **WO-A-20/04072205**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 838 671 B1

Printed by Jouve, 75001 PARIS (FR)

## Description

FIELD OF THE INVENTION

The present invention relates to carbazole compounds and a semiconducting material comprising carbazole compounds. It also relates to a process for the preparation of carbazole compounds, as well as to the use thereof as a semiconducting material, in particular as a host matrix for phosphorescent emitters.

BACKGROUND OF THE INVENTION

As phosphorescent emitters in OLEDs (organic light emitting diodes), metal complexes can be used. The combination of a semiconducting material with suitable metal complexes can give rise to an increase in the efficiency of light-emitting diodes, because the metal complexes can harvest both singlet *and* triplet excited states due to their ability to mix the singlet and triplet state via the heavy atom effect. By this mixing a state is generated that is emissive so that 100% of the excited states (i.e. both fluorescence and phosphorescence) in an OLED can be used for the emission of light (see Fig. 1).

The semiconducting material is called the host matrix for the phosphorescent emitters and is needed to form thin layers of the phosphorescent material in OLEDs and to transport charges. The host matrix for phosphorescent emitters has to fulfil - besides the usual requirements known already from the use of fluorescent emitters - the additional condition that the triplet energy gap of the host has to be higher than that of the guest. That is, in order to provide efficient phosphorescence from the metal complex, the lowest excited state (triplet state, $T_{HOST}$ in Fig. 1) of the host matrix has to be higher in energy than the lowest emitting state of the metal complex ($T_{GUEST}$ in Fig. 1). This requirement arises because energy will always reside on the *lowest excited state* of a system. Since the phosphorescent metal complex shall be the emitter, the lowest excited state has to be on the metal complex and not on the host matrix.

WO 04/072205 discloses carbazole derivatives for use as host material for small-molecule OLEDs based on derivatization at the phenyl rings of the carbazole unit. The carbazole is either connected to other carbazoles to form carbazole dimers and trimers or to fluorene and oxadiazole to form mixed compounds.

The energy level of the triplet state of the carbazole derivatives is just high enough to accommodate a sky-blue phosphorescent emitter. The triplet energy of the carbazole hosts is at about 2.75 eV (450 nm) for all derivatives. That limits the applicability of the host to blue phosphorescent emitters of an energy of 2.725 eV (455 nm), that is to phosphorescent emitters that are at least the room temperature energy kT below the triplet energy level of the host, but more realistically seen to blue phosphorescent emitters of energy of 2.675 eV (464 nm) that are three times kT below the triplet energy level of the host. That means that the triplet energy level of the carbazole derivatives is too low to accommodate saturated blue phosphorescent emitters (at about 2.8 eV, 443 nm) efficiently.

For most applications a (saturated) blue color is needed. Therefore there is a need for hosts with triplet energy levels > 2.875 eV that can accommodate saturated phosphorescent triplet emitters in the range of 2.85 eV to 2.8 eV efficiently.

It is not straightforward to find a suitable host for high-energy triplet emitters for OLEDs. Materials with a large HOMO-LUMO energy gap will automatically have a high triplet energy. However, these materials will also suffer from charge injection problems due to misalignment of the HOMO and/or LUMO level with the Fermi levels of the electrodes (or charge injection layers). So at the same time, the triplet energy level has to be increased and the oxidation/reduction potential must remain in a regime where easy charge injection from common contacts is possible.

It is known in the art that an energy level is increased upon localization of the wavefunction of a molecule. For example, structures that use a methyl group to twist a biphenyl, which increases the triplet energy level, are known in literature (4,4'-bis(9-carbazolyl)-2,2'-dimethyl-biphenyl (CDBP), Tokito, S.; Iijima, T.; Suzuri, Y.; Kita, H.; Tsuzuki, T.; Sato, F. Appl. Phys. Lett. 2003, 83, 569). However, prior art does not reveal how to obtain high triplet energy levels in combination with a constant oxidation/reduction potential.

It would of course be very advantageous to be able to provide chemical compounds having the desired characteristics.

SUMMARY OF THE INVENTION

An aim of the present invention is to be able to increase the triplet energy level of hosts for OLEDs to values sufficiently high to accommodate saturated blue phosphorescent emitters, keeping the oxidation potential at values where charge injection of positive charge carriers is easily possible.

This aim is achieved by a carbazole compound having the general formula (I)

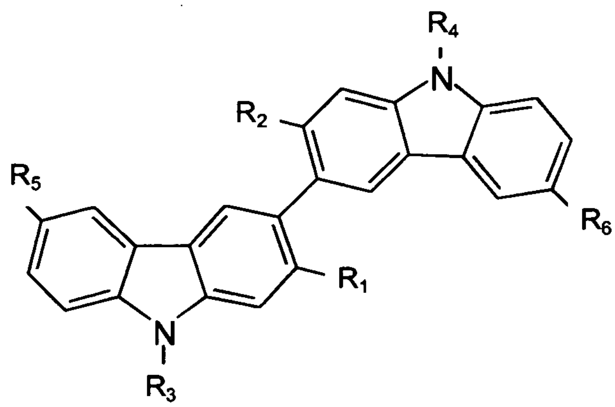

(I)

wherein

$-R_1$, and $-R_2$ are, the same or different at each occurrence, $-OR^{41}$, $-OR^{42}$, $-SR^{41}$, $-SR^{42}$, $-NR^{41}R^{45}$, or $-NR^{42}R^{45}$;
$-R_3$, and $-R_4$ are, the same or different at each occurrence, $R^{41}$, or $R^{42}$, with
$R^{41}$ being $C_1$-$C_{20}$ cyclic or acyclic straight or branched alkyl, optionally interrupted one or more times with -O-, -OC(=O)-, -C(=O)O-, -S-, secondary nitrogen, tertiary nitrogen, quaternary nitrogen, $-CR^{45}=CR^{46}-$, -C≡C-, -C(=O)-, $-C(=O)NR^{45}-$, $-NR^{45}C(=O)-$, -S(=O)-, $-S(=O)_2-$, or $-X^6-$, and/or substituted one or more times with $R^{42}$, $R^7$, or $R^8$;
$R^{42}$ being $C_5$-$C_{30}$ aryl in which, optionally, one or more of the aromatic carbon atoms are replaced with N, O or S, and, optionally, one or more of the aromatic carbon atoms carry a group $R^{41}$, $R^7$, or $R^8$;
$R^7$ being -CN, $-CF_3$, -CSN, $-NH_2$, $-NO_2$, -NCO, -NCS, -OH, -F, $-PO_2$, $-PH_2$, -SH, -Cl, -Br, or -I;
$R^8$ being $-C(=O)R^{45}$, $-C(=O)OR^{45}$, $-C(=O)NR^{45}R^{46}$, $-NHR^{45}$, $-NR^{45}R^{46}$, $-N^{(+)}R^{45}R^{46}R^{47}$, $-NC(=O)R^{45}$, $-OR^{45}$, $-OC(=O)R^{45}$, $-SR^{45}$, $-S(=O)R^{45}$, or $-S(=O)_2R^{45}$;
$R^{45}$, $R^{46}$, and $R^{47}$ being, the same or different at each occurrence, H, $R^{41}$, or $R^{42}$;
$X^6$ being $C_4$-$C_{30}$ arylene in which, optionally, one or more of the aromatic carbon atoms are replaced with N, O, or S, and, optionally, one or more of the aromatic carbon atoms carry a group $R^{41}$, $R^7$, or $R^8$; and
$-R_5$, and $-R_6$ are, the same or different at each occurrence, H, $R^{7'}$, $R^{41'}$, or $R^{42'}$, with
$R^{41'}$ being $C_1$-$C_{20}$ cyclic or acyclic straight or branched alkyl, optionally interrupted one or more times with -O-, -OC(=O)-, -C(=O)O-, -S-, secondary nitrogen, tertiary nitrogen, quaternary nitrogen, $-CR^{45'}=CR^{46'}-$, -C≡C-, -C(=O)-, $-C(=O)NR^{45'}-$, $-NR^{45'}C(=O)-$, -S(=O)-, $-S(=O)_2-$, or $-X^{6'}-$; and/or substituted one or more times with $R^{42'}$, $R^{7'}$, or $R^{8'}$;
$R^{42'}$ being $C_5$-$C_{30}$ aryl, in which one or more of the aromatic carbon atoms in ortho position carry a group $R^{41'}$, $R^{45'}$, $R^{7'}$, or $R^{8'}$, and, optionally, one or more of the aromatic carbon atoms are replaced with N, O or S, and, optionally, one or more of the aromatic carbon atoms carry a group $R^{41'}$, $R^{7'}$, or $R^{8'}$;
$R^{7'}$ being -CN, $-CF_3$, -CSN, $-NH_2$, $-NO_2$, -NCO, -NCS, -OH, -F, $-PO_2$, $-PH_2$, -SH, -Cl, -Br, -I, or $-B(OR^{41'})(OR^{45'})$;
$R^{8'}$ being $-C(=O)R^{45'}$, $-C(=O)OR^{45'}$, $-C(=O)NR^{45'}R^{46'}$, $-NHR^{45'}$, $-NR^{45'}R^{46'}$, $-N^{(+)}R^{45'}R^{46'}R^{47'}$, $-NC(=O)R^{45'}-$, $-OR^{45'}$, $-OC(=O)R^{45'}$, $-SR^{45'}$, $-S(=O)R^{45'}$, or $-S(=O)_2R^{45'}$;
$R^{45'}$, $R^{46'}$, $R^{47'}$ being, the same or different at each occurrence, H, $R^{41'}$, or $R^{42'}$;
$X^{6'}$ being $C_4$-$C_{30}$ arylene in which, optionally, one or more of the aromatic carbon atoms are replaced with N, O, or S, and, optionally, one or more of the aromatic carbon atoms carry a group $R^{41'}$, $R^{7'}$, or $R^{8'}$.

The $R_1$ and $R_2$ groups cause the biphenyl structure to twist, i.e. the carbazoles rotate around the single bond between the two carbazoles, so that the two carbazoles are not in-plane any more. Thereby, the wavefunction is no longer completely delocalised over both carbazoles, and the triplet energy level is increased, at the same time as the oxidation potential remains constant.

For example, $R_1$ and $R_2$ may be $-OR^{41}$, wherein $R^{41}$ is defined above. For example $R_1$ and $R_2$ are methyloxy ($-OCH_3$), or straight or branched chain decyloxy ($-OC_{10}H_{21}$). By attaching longer chains the triplet energy level increases.

For example, $R_3$ and $R_4$ may be $R^{41}$, wherein $R^{41}$ is defined above. For example, $R_3$ and $R_4$ are straight or branched chain decyl ($-C_{10}H_{21}$).

For example, $R_5$ and $R_6$ may be H, $R^{7'}$, or $R^{42'}$, wherein $R^{7'}$, and $R^{42'}$ is defined above. For example, $R_5$ and $R_6$ may be Br or ortho-methoxyphenyl.

Further, the invention relates to a semiconducting material comprising a compound of formula I, and to an electroluminescent device comprising such a semiconducting material. The semiconducting material comprising compounds of formula I may also be combined with phosphoresent emitters, e.g. metal complexes.

The invention also relates to a process for the preparation of a compound of formula I, as well as to the use of a compound of formula I as a semiconducting material, in particular as a host matrix for phosphorescent emitters.

BRIEF DESCRIPTION OF THE DRAWINGS

Fig. 1 is an illustration of the condition that the triplet energy level of the host polymer has to be higher than that of the phosphorescent emitter. On the host polymer (left), singlet and triplet excited states are generated. If the energy level of the phosphorescent emitter (right) is below that of the host polymer, the phosphorescent emitter can harvest singlet and triplet excitations from the host polymer, thereby increasing the efficiency of an organic light emitting diode to 100 %.

DESCRIPTION OF PREFERRED EMBODIMENTS

The following formula shows the chemical structure of a basic building block of the carbazole derivatives: [3,3']-bicarbazolyl. The approximate position of the triplet wavefunction is indicated with a dashed line.

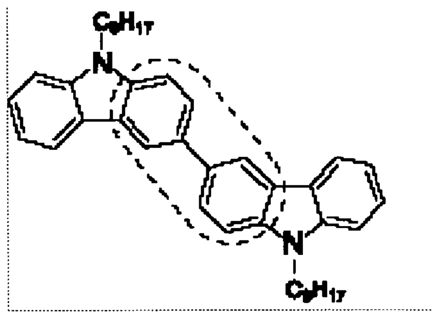

For [3,3']-bicarbazolyl, the triplet energy value is 2.75 eV, while the triplet energy of the corresponding monomer, carbazole, is 3.05 eV. In [3,3']-bicarbazolyl, the triplet exciton is more delocalized than in carbazole, and the triplet exciton is predominantly delocalized over the biphenyl structure that is shared between the two carbazole units.

In general it can be said, that the more delocalized the triplet wavefunction is, the lower the triplet energy level will be. Thus, to be able to increase the triplet energy level of [3,3']-bicarbazolyl, the localization of the triplet wavefunction on the basic building blocks of the carbazole derivatives needs to be affected. To increase the triplet level, the delocalization of the triplet wavefunction over the biphenyl structure has to be decreased. This can for example be done by twisting the biphenyl structure.

A very important aspect is that by twisting the biphenyl structure the oxidation potential of the carbazole building block must not increase. A high oxidation potential generally means a high barrier for hole injection from commonly used anodes such as indium tin oxide. In such a case additional hole injection and transporting layers have to be introduced thereby complicating the device architecture.

It is not straightforward to meet the requirement of a low oxidation potential in twisted carbazoles since by twisting the carbazole dimer the two constituents are decoupled and the dimer approaches spectroscopically a monomer (as is desired for a high triplet energy level). But a carbazole monomer has a considerably higher oxidation potential than a carbazole dimer (1.14 vs 0.86 V).

This dilemma was solved according to this invention by inducing the twist in the bicarbazole with electron donating groups such as methoxy groups. At the same time as the biphenyl structure is twisted and the triplet level is increased, the electron density is also increased at the ring structure which leads to a decrease in the oxidation potential.

By "twisting" is meant a rotation of the carbazoles around the single bond between the two carbazoles, so that the two carbazoles are not in-plane any more. That has the effect that the wavefunction cannot be delocalised over both carbazoles, which increases the triplet level.

Thus, the present inventors have succeeded in increasing the triplet energy level of hosts for OLEDs to values sufficiently high to accommodate saturated blue phosphorescent emitters, and at the same time keeping the oxidation potential at values where charge injection of positive charge carriers is easily possible.

The carbazole compound according to the present invention has the general formula (I)

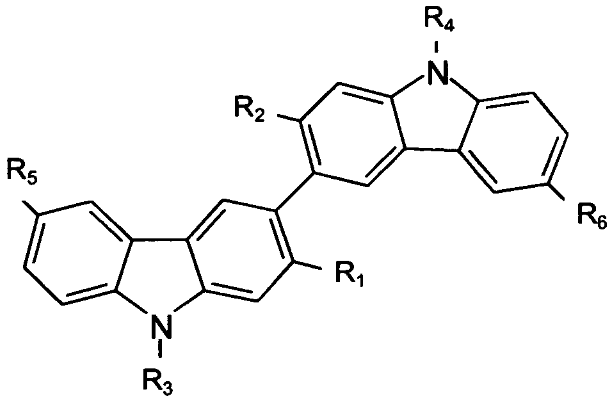

(I)

wherein

$-R_1$, and $-R_2$ are, the same or different at each occurrence, $-OR^{41}$, $-OR^{42}$, $-SR^{41}$, $-SR^{42}$,$-NR^{41}R^{45}$, or $-NR^{42}R^{45}$ ;
$-R_3$, and $-R_4$ are, the same or different at each occurrence, $R^{41}$, or $R^{42}$, with
$R^{41}$ being $C_1$-$C_{20}$ cyclic or acyclic straight or branched alkyl, optionally interrupted one or more times with -O-, -OC(=O)-, -C(=O)O-, -S-, secondary nitrogen, tertiary nitrogen, quaternary nitrogen, $-CR^{45}=CR^{46}-$, -C≡C-, -C(=O)-, -C(=O)$NR^{45}$-, - $NR^{45}$C(=O)-, -S(=O)-, $-S(=O)_2-$, or $-X^6-$, and/or substituted one or more times with $R^{42}$, $R^7$, or$R^8$;
$R^{42}$ being $C_5$-$C_{30}$ aryl in which, optionally, one or more of the aromatic carbon atoms are replaced with N, O or S, and, optionally, one or more of the aromatic carbon atoms carry a group $R^{41}$, $R^7$, or $R^8$;
$R^7$ being -CN, $-CF_3$, -CSN, $-NH_2$, $-NO_2$, -NCO, -NCS, -OH, -F, $-PO_2$, $-PH_2$, -SH, -Cl, -Br, or -I;
$R^8$ being $-C(=O)R^{45}$, $-C(=O)OR^{45}$, $-C(=O)NR^{45}R^{46}$, $-NHR^{45}$,$-NR^{45}R^{46}$, $-N^{(+)}R^{45}R^{46}R^{47}$, $-NC(=O)R^{45}-$, $-OR^{45}$, $-OC(=O)R^{45}$, $-SR^{45}$, $-S(=O)R^{45}$, or - $S(=O)_2R^{45}$;
$R^{45}$, $R^{46}$, and $R^{47}$ being, the same or different at each occurrence, H, $R^{41}$, or $R^{42}$;
$X^6$ being $C_4$-$C_{30}$ arylene in which, optionally, one or more of the aromatic carbon atoms are replaced with N, O, or S, and, optionally, one or more of the aromatic carbon atoms carry a group $R^{41}$, $R^7$, or $R^8$; and
$-R_5$, and $-R_6$ are, the same or different at each occurrence, H, $R^{7'}$, $R^{41'}$, or $R^{42'}$, with
$R^{41'}$ being $C_1$-$C_{20}$ cyclic or acyclic straight or branched alkyl, optionally interrupted one or more times with -O-, -OC(=O)-, -C(=O)O-, -S-, secondary nitrogen, tertiary nitrogen, quaternary nitrogen, $-CR^{45'}=CR^{46'}-$, -C≡C-, -C(=O)-, -C(=O)$NR^{45'}$-, - $NR^{45'}$C(=O)-, -S(=O)-, $-S(=O)_2-$, or $-X^{6'}-$; and/or substituted one or more times with $R^{42'}$, $R^{7'}$, or $R^{8'}$;
$R^{42'}$ being $C_5$-$C_{30}$ aryl, in which one or more of the aromatic carbon atoms in ortho position carry a group $R^{41'}$, $R^{45'}$, $R^{7'}$, or $R^{8'}$, and, optionally, one or more of the aromatic carbon atoms are replaced with N, O or S, and, optionally, one or more of the aromatic carbon atoms carry a group $R^{41'}$, $R^{7'}$, or $R^{8'}$;
$R^{7'}$ being -CN, $-CF_3$, -CSN, $-NH_2$, $-NO_2$, -NCO, -NCS, -OH, -F, - $PO_2$, $-PH_2$, -SH, -Cl, -Br, -I, or $-B(OR^{41'})(OR^{45'})$;
$R^{8'}$ being $-C(=O)R^{45'}$, $-C(=O)OR^{45'}$, $-C(=O)NR^{45'}R^{46'}$, $-NHR^{45'}$, - $NR^{45'}R^{46'}$, $-N^{(+)}R^{45'}R^{46'}R^{47'}$, $-NC(=O)R^{45'}-$, $-OR^{45'}$, $-OC(=O)R^{45'}$, $-SR^{45'}$, $-S(=O)R^{45'}$, or - $S(=O)_2R^{45'}$;
$R^{45'}$, $R^{46'}$, $R^{47'}$ being, the same or different at each occurrence, H, $R^{41'}$, or $R^{42'}$;
$X^{6'}$ being $C_4$-$C_{30}$ arylene in which, optionally, one or more of the aromatic carbon atoms are replaced with N, O, or S, and, optionally, one or more of the aromatic carbon atoms carry a group $R^{41'}$, $R^{7'}$, or $R^{8'}$.

Although $R_1$ and $R_2$ are placed at the 2 and 2' position, respectively, of the carbazole unit in the disclosed embodiments, it is to be understood that $R_1$ and $R_2$ could also be placed in the 4 and 4' position.

Likewise, although $R_5$ and $R_6$ are placed at the 6 and 6' position, respectively, of the carbazole unit in the disclosed embodiments, it is to be understood that $R_5$ and $R_6$ could also be placed in the 5 and 5' position, the 6 and 6' position, the 7 and 7' position, or the 8 and 8' position.

The introduction of electron donating groups, like alkyloxy/aryloxy, alkylthio/arylthio and dialkylamine/diarylamine, at the $R_1$ and $R_2$ positions, influence the HOMO level such that hole injection remains easy.

$R_1$ and $R_2$ are substituents having a total number of non-hydrogen atoms less than 40. Examples of $R_1$ and $R_2$ groups according to the invention are methoxy, ethoxy, propoxy, hexyloxy, octyloxy, nonyloxy and decyloxy. Isomers, such as isopropyloxy, and other branched or cyclic alkoxies may also be used. In particular, methoxy and decyloxy may be used in the compound according to the invention.

Examples of $R_3$ and $R_4$ groups according to the invention are methyl, ethyl, propyl, butyl, pentyl, hexyl, heptyl, octyl, nonyl, and decyl. Isomers, such as isopropyl, and other branched or cyclic alkyl groups may also be used. In

particular, decyl may be used in the compound according to the invention.

Examples of $R_5$ and $R_6$ groups are halogens, i.e. F, Cl, Br, I, and also borolane. When $R_5$ and $R_6$ are aryl groups, they are substituted at least in the ortho position, in order to obtain the desired twist. For example, ortho substituted phenyl, in particular orthomethoxyphenyl, may be used in the compound according to the invention.

The compounds according to the invention may also be used in combination with other compounds, such as e.g. electron transporting molecules, hole blocking molecules, exciton blocking molecules, electron blocking molecules, additional injection layers for electrons or holes, and additional hole transporting layers.

The compounds according to the invention may be used as a semiconducting material, in particular as a host matrix for phosphorescent emitters. Examples of phosphorescent emitters, i.e. phosphorescent acceptor compounds, are metal complexes, such as rare earth and lanthanide metal complexes, e.g. iridium metal complexes.

By the introduction of phosphorescent emitters in the semiconducting material, both phosphorescence *and* fluorescence, i.e. all excited states formed in an OLED may be used The phosphorescent emitters can harvest *both* the triplet *and* the singlet excitations formed in the emissive layer and thereby use all excited states for the emission of light. The device efficiency is so considerably increased.

The semiconducting material according to the invention may be used in electroluminescent devices, e.g. OLEDs, light emitting electrochemical cells or in other organic electronic devices, such as transistors and memory devices.

The invention will now be further explained in the following examples. These examples are only intended to illustrate the invention and should in no way be considered to limit the scope of the invention.

EXAMPLES

In table 1 a non twisted structure and twisted biphenyl structures of a basic building block of the carbazole derivatives are shown together with the triplet energy levels. The twisting is achieved by attaching side chains at the 2 and 2' position of the [3,3']-bicarbazolyl. It can clearly be seen that the triplet energy level of the twisted structures is considerably higher than the triplet energy level of the untwisted structure.

As can be seen from table 1, the oxidation potential $(E_{1/2}^{ox})$ of the twisted carbazoles is (within the error margins of the experiment) the same as $E_{1/2}^{ox}$ of the untwisted carbazoles.

The triplet levels where determined with phosphorescence measurements. The phosphorescence spectra were obtained on highly diluted (about 1 mg/1) solutions in methyl-THF, which gives a clear glass at 77 K. The emission spectra at 77 K were recorded with an Edinburgh 900 spectrofluorometer. Non-gated and gated spectra were recorded to discriminate the phosphorescence from fluorescence. The gate delay was 500 μs with a gate width of 9 ms. The highest energy peak in the phosphorescence spectrum was taken for the $S_0^{v=0} \leftarrow T_1^{v=0}$ transition.

The oxidation potential was determined with cyclic voltammetry (CV) measurements. CV measurements were recorded in dichloromethane, with 1 M tetrabutylammonium hexafluorophosphate as supporting electrolyte. The working electrode was a platinum disc (0.2 $cm^2$), the counter electrode was a platinum plate (0.5 $cm^2$), and a saturated Ag/AgCl was used as reference electrode, calibrated against a $Fc/Fc^+$ couple.

Table 1

| Example No | Structure | Triplet level (eV) | $E_{1/2}^{ox}$ (V) |
|---|---|---|---|
| non twisted structure | 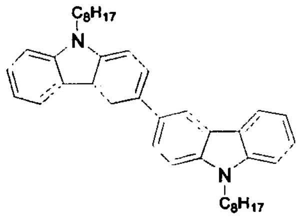 | 2.75 | 0.86 |

(continued)

| Example No | Structure | Triplet level (eV) | $E^{ox}_{1/2}$ (V) |
|---|---|---|---|
| 24 | 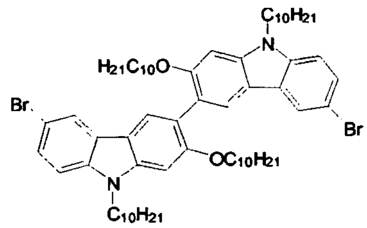 | 2.89 | 0.89 |
| 15 | 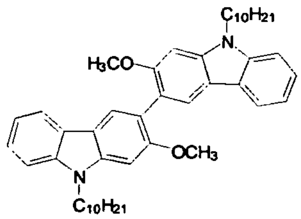 | 2.97 | 0.82 |
| 25 | 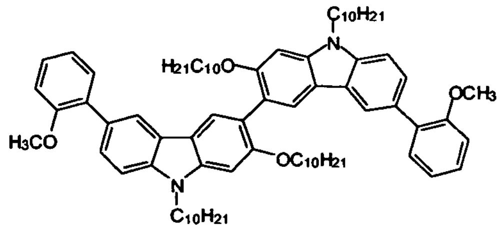 | 2.96 | 0.77 |

Synthesis examples

*Synthesis example 1*

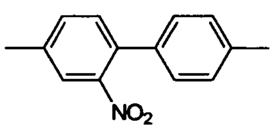

4,4'-dimethyl-2-nitro-1,1'-biphenyl

A flask, covered with aluminium foil, was charged with 5.0 g (19 mmol) 4-iodo-3-nitrotoluene, 4.14 g (19 mmol) 4-(4,4,5,5-tetramethyl-1,3,2-dioxaborolyl)toluene, 20 ml toluene and 20 ml 2 M potassium carbonate (aq). The mixture was evacuated and charged with argon for three times, after which 2 mol% $Pd(PPh_3)_4$ was added. Evacuation and filling with argon was repeated once and the mixture was stirred for 60 hours at reflux temperature. The mixture was allowed to cool to room temperature, the organic layer was separated, dried ($MgSO_4$), filtered and concentrated. After column chromatography ($SiO_2$, hexane/dichloromethane, 60/40, v/v) 4.3 g of product was obtained.
$^1$H NMR ($CDCl_3$): δ 7.64 (d, *J*=1.5 Hz, 1H), 7.41 (dd, *J*=1.5 Hz, *J*=8 Hz, 1H), 7.32 (d, *J*=8 Hz, 1H), 7.26-7.19 (m, 4H), 2.47 (s, 3H), 2.40 (s, 3H).
$^{13}$C NMR ($CDCl_3$): δ 149, 138, 138, 134, 133, 133, 132, 129, 128, 124, 21, 21.

*Synthesis example 2*

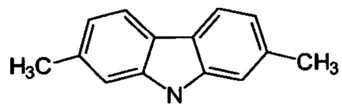

2,7-dimethylcarbazole

4.3 g (19 mmol) 4,4'-dimethyl-2-nitro-1,1'-biphenyl in 15 ml triethylphosphite is refluxed during 16 hours in argon atmosphere. The mixture was allowed to cool to room temperature, upon which the product precipitated. Filtration and washing with methanol gave 0.88 g (24 %) of the white solid.
$^1$H NMR (DMSO-$d_6$): δ 11.0 (s, 1H), 7.92 (d, *J*=8 Hz, 2H), 7.25 (s, *J*=1.5 Hz, 2H), 6.96 (d, *J*=1.5 Hz, *J*=8 Hz, 2H), 2.41 (s, 6H).
$^{13}$C NMR (DMSO-$d_6$): δ 140, 134, 120, 120, 119, 111, 21.
mp: 292 °C.

*Synthesis example 3*

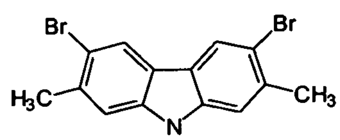

3,6-dibromo-2,7-dimethylcarbazole

In a flask, covered with aluminum foil, a stirred solution of 0.5 g (2.6 mmol) 2,7-dimethylcarbazole in 10 ml tetrahydrofuran was cooled to 0 °C. 0.86 g (4.8 mmol) N-bromosuccinimide was added in small portions. The mixture was allowed to warm to room temperature overnight. The THF was evaporated and the solid was used without further purification.
$^1$H NMR (DMSO-$d_6$): δ 11.38 (s, 1H), 8.40 (s, 1H), 7.46 (s, 1H), 2.41 (s, 6H).

*Synthesis example 4*

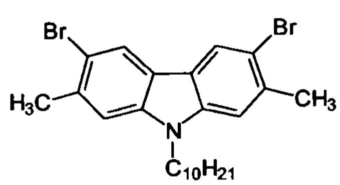

3,6-dibromo-2,7-dimethyl-9-(3,7-dimethyloctyl)carbazole

To a stirred solution of 1.6 g (4.6 mmol) 3,6-dibromo-2,7-dimethylcarbazole and 26 mg benzyltriethylammoniumchloride in 10 ml toluene was added dropwise 2.1 g 50 w% NaOH (aq). Afterwards 1.5 g (6.8 mmol) 3,7-dimethyloctylbromide was added dropwise. After complete addition the reaction mixture was heated to reflux during 48 hours.

The organic layer was separated, washed with water, dried over $MgSO_4$, filtered and concentrated. After column chromatography ($SiO_2$, hexane/dichloromethane, 80/20, v/v) followed by crystallisation (dichloromethane/methanol) 1.0 g (44 %) of product was obtained.
$^1$H NMR ($CDCl_3$): δ 8.12 (s, 1H), 7.19 (s, 1H), 4.17 (t, *J*=8 Hz, 2H), 2.58 (s, 6H), 1.84-1.10 (m, 10H), 1.02 (d, *J*=6.5 Hz, 3H), 0.85 (d, *J*=6.5 Hz, 6H),
$^{13}$C NMR ($CDCl_3$): δ.140, 135, 124, 122, 115, 110, 41, 39, 37, 35, 31, 28, 25, 24, 23, 23, 20. mp: 61 ˚C.

*Synthesis example 5*

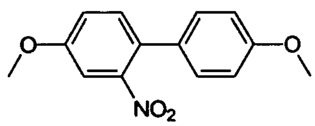

4,4'-dimethoxy-2-nitro-1,1'-biphenyl

A flask, covered with aluminium foil, was charged with 14.9 g (64 mmol) 4-bromo-3-nitroanisole, 18 g (77 mmol) 4-(4,4,5,5-tetramethyl-1,3,2-dioxaborolyl)anisole, 70 ml toluene and 70 ml 2 M potassium carbonate (aq). The mixture was evacuated and charged with argon for three times, after which 2 mol% $Pd(PPh_3)_4$ was added. Evacuation and filling with argon was repeated once and the mixture was stirred for 60 hours at reflux temperature. The mixture was allowed to cool to room temperature, the organic layer was separated, dried ($MgSO_4$), filtered and concentrated. After column chromatography ($SiO_2$, hexane/dichloromethane, 50/50, v/v) 12.0 g (72 %) of product was obtained.
$^1$H NMR ($CDCl_3$): δ 7.33 (s, *J*=1.5 Hz, 1H), 7.32 (d, *J*=8 Hz, 1H), 7.20 (d, *J*=8 Hz, 2H), 7.12 (dd, *J*=1.5 Hz, *J*=8 Hz, 1H), 6.93 (d, .*J*=8 Hz, 2H), 3.88 (s, 3H), 3.83 (s, 3H).
$^{13}$C NMR ($CDCl_3$): δ 159, 159, 149, 132, 129, 129, 128, 119, 114, 109, 56, 55.
mp: 138˚C.

*Synthesis example 6*

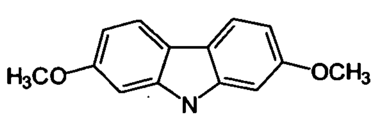

2,7-dimethoxycarbazole

10 g (38.6 mmol) 4,4'-dimethoxy-2-nitro-1,1'-biphenyl in 35 ml triethylphosphite is refluxed during 16 hours in argon atmosphere. The mixture was allowed to cool to room temperature, upon which the product precipitates. Filtration and washing with methanol gave 6.5 g (74 %) of a white solid.
$^1$H NMR (DMSO-$d_6$): δ 11.00 (s, 1H), 7.85 (d, *J*=8 Hz, 2H), 6.94 (d, *J*=1.5 Hz, 2H), 6.74 (dd, *J*=1.5 Hz, *J*=8 Hz, 2H), 3.82 (s, 6H).
$^{13}$C NMR (DMSO-$d_6$): δ 157, 141, 119, 116, 107, 94, 55.
mp: 285 ˚C.

*Synthesis example 7*

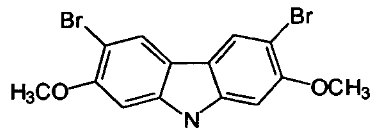

3,6-dibromo-2,7-dimethoxycarbazole

In a flask, covered with aluminum foil, a stirred solution of 1.48 g (6.5 mmol) 2,7-dimethoxycarbazole in 60 ml tetrahydrofuran was cooled to 0 ˚C. 2.3 g (13 mmol) N-bromosuccinimide was added in small portions. The mixture was allowed to warm to room temperature overnight. THF was evaporated and the product was used without further purification.
$^{1}H$ NMR (DMSO-$d_6$): δ 11.30 (s, 1H), 8.30 (s, 2H), 7.13 (s, 2H), 3.90 (s, 6H).
$^{13}C$ NMR (DMSO-$d_6$): δ 153, 140, 123, 116, 102, 94, 56.

*Synthesis example 8*

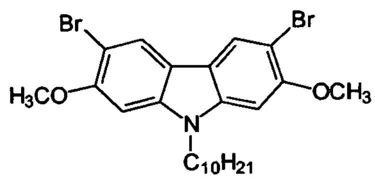

3,6-dibromo-9-(3,7-dimethyloctyl)-2,7-dimethoxycarbazole

To a stirred solution of 2.5 g (6.5 mmol) 3,6-dibromo-2,7-dimethoxycarbazole and 40 mg benzyltriethylammoniumchloride in 10 ml toluene was added dropwise 3.8 g 50 w% NaOH (aq). Afterwards 1.7 g (7.7 mmol) 3,7-dimethyloctylbromide was added dropwise. After complete addition the reaction mixture was heated to reflux during 16 hours. The organic layer was separated, washed with water, dried over $MgSO_4$, filtered and concentrated. After column chromatography ($SiO_2$, hexane/dichloromethane/$Et_3N$, 80/20/1, v/v/v), followed by crystallisation (dichloromethane/ethanol) 2.1 g (61 %) of a white solid was obtained.
$^{1}H$ NMR ($CDCl_3$): δ 8.10 (s, 2H), 6.84 (s, 2H), 4.26 (t, *J*=8 Hz, 2H), 4.03 (s, 6H), 1.98-1.150 (m, 10H), 1.07 (d, *J*=6.5 Hz, 3H), 0.88 (d, *J*=6.5 Hz, 6H).
$^{13}C$ NMR ($CDCl_3$): δ 154, 140, 124, 117, 103, 92, 56, 41, 39, 37, 35, 31, 28, 25, 23, 23, 20. mp: 113 ˚C.

*Synthesis example 9*

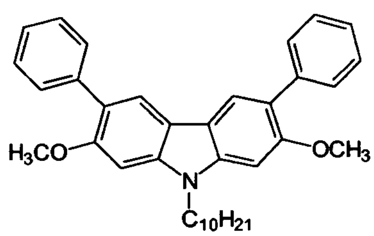

9-(3,7-dimethyloctyl)-2,7-dimethoxy-3,6-diphenylcarbazole

A flask, covered with aluminum foil, was charged with 0.5 g (1 mmol) 3,6-dibromo-9-(3,7-dimethyloctyl)-2,7-dimethoxycarbazole, 0.5 g (2.5 mmol) (4,4,5,5-tetramethyl-1,3,2-dioxaborolyl)benzene, 10 ml toluene and 10 ml 2 M potassium carbonate (aq). The mixture was evacuated and charged with argon for three times, after which 2 mol% Pd$(PPh_3)_4$ was added. Evacuation and filling with argon was repeated once and the mixture was stirred for 16 hours at reflux temperature. The mixture was allowed to cool to room temperature, the organic layer was separated, dried ($MgSO_4$), filtered and concentrated. After column chromatography ($SiO_2$, hexane/dichloromethane, 60/40, v/v) 0.4 g (81 %) of product was obtained.
$^1$H NMR ($CDCl_3$): δ 7.92 (s, 2H), 7.63 (dd, *J*=1.5 Hz, *J*=8 Hz, 4H), 7.45 (t, *J*=8 Hz, 4H), 7.33 (dt, *J*=1.5 Hz, *J*=8 Hz, 2H), 6.92 (s, 2H), 4.30 (t, *J*=8 Hz, 2H), 3.93 (s, 6H), 1.98-1.15 (m, 10H), 1.07 (d, *J*=6.5 Hz, 3H), 0.88 (d, *J*=6.5 Hz, 6H).
$^{13}$C NMR ($CDCl_3$): δ 155, 141, 140, 130, 128, 126, 124, 122, 117, 92, 56, 41, 39, 37, 35, 31, 28, 25, 23, 23, 20.
mp: 119°C.

*Synthesis example 10*

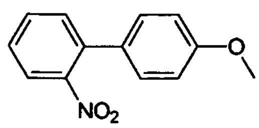

4'-methoxy-2-nitro-1,1'-biphenyl

A flask, covered with aluminium foil, was charged with 10.7 g (53 mmol) 1-bromo-2-nitrobenzene, 14.9 g (64 mmol) 4-(4,4,5,5-tetramethyl-1,3,2-dioxaborolyl)anisole, 70 ml toluene and 70 ml 2 M potassium carbonate (aq). The mixture was evacuated and charged with argon for three times, after which 2 mol% Pd$(PPh_3)_4$ was added. Evacuation and filling with argon was repeated once and the mixture was stirred for 48 hours at reflux temperature. The mixture was allowed to cool to room temperature, the organic layer was separated, dried ($MgSO_4$), filtered and concentrated. After column chromatography ($SiO_2$, hexane/dichloromethane, 60/40, v/v) 8.5 g (70 %) of product was obtained.
$^1$H NMR ($CDCl_3$): δ 7.84 (dd, *J*=1.5 Hz, *J*=8 Hz, 1H), 7.62 (dt, *J*=1.5 Hz, *J*=8 Hz, 1H), 7.48 (dd, *J*=1.5 Hz, *J*=8 Hz, 1H), 7.46 (dt, *J*=1.5 Hz, *J*=8 Hz, 1H), 7.29 (d, *J*=8 Hz, 2H), 7.00 (d, *J*=8 Hz, 2H), 3.82 (s, 3H).
$^{13}$C NMR ($CDCl_3$): δ 159, 149, 136, 132, 132, 129, 129, 128, 124, 114, 55.

*Synthesis example 11*

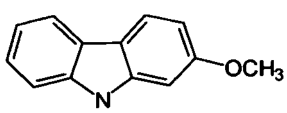

2-methoxycarbazole

8.36 g (36.7 mmol) 4'-methoxy-2-nitro-1,1'-biphenyl in 40 ml triethylphosphite is refluxed during 16 hours in argon atmosphere. The mixture was allowed to cool to room temperature, upon which the product precipitates. Filtration and washing with methanol gave 6.67 g (93 %) of a white solid
$^1$H NMR (DMSO-$d_6$): δ11.10 (s, 1H), 8.00 (dd, *J*=1.5 Hz, *J*=8 Hz, 1H), 7.8 (d, *J*=8 Hz, 1H), 7.44 (dd, *J*=1.5 Hz, *J*=8 Hz, 1H), 7.30 (dt, *J*=1.5 Hz, *J*=8 Hz, 1H), 7.12 (dt, *J*=1.5 Hz, *J*=8 Hz, 1H), 6.98 (d, *J*=1.5 Hz, 1H), 6.78 (dd, *J*=1.5 Hz, *J*=8 Hz, 1H), 3.83 (s, 3H).
$^{13}$C NMR (DMSO-$d_6$): δ 158, 141, 140, 124, 123, 121, 119, 118, 116, 111, 108, 94, 55.

mp: 239 ˚C.

*Synthesis example 12*

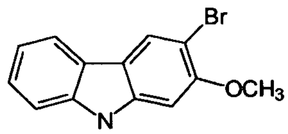

3-bromo-2-methoxycarbazole

In a flask, covered with aluminium foil, a stirred solution of 6.62 g (33.6 mmol) 2-methoxycarbazole in 150 ml tetrahydrofuran was cooled to 0 ˚C. 5.38 g (30.2 mmol) N-bromosuccinimide was added in small portions. The mixture was allowed to warm to room temperature overnight. THF was evaporated and the product was used without further purification.
$^1$H NMR (DMSO-$d_6$): δ 11.30 (s, 1H), 8.34 (s, 1H), 8.04 (dd, *J*=1.5 Hz, *J*=8 Hz, 1H), 7.46 (dd, *J*=1.5 Hz, *J*=8 Hz, 1H), 7.33 (dt, *J*=1.5 Hz, *J*=8 Hz, 1H), 7.14 (dt, *J*=1.5 Hz, *J*=8 Hz, 1H), 7.14 (s, 1H), 3.93 (s, 3H).
$^{13}$C NMR (DMSO-$d_6$): δ 153, 140, 139, 124, 124, 122, 120, 119, 116, 110, 101, 95, 56.

*Synthesis example 13*

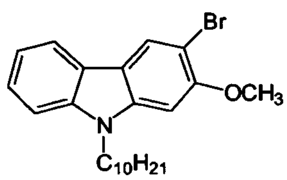

3-bromo-9-(3,7-dimethyloctyl)-2-methoxycarbazole

To a stirred solution of 7.16 g (26 mmol) 3-bromo-2-methoxycarbazole and 0.17 g benzyltriethylammoniumchloride in 25 ml toluene was added dropwise 15 g 50 w% NaOH (aq). Afterwards 6.9 g (31 mmol) 3,7-dimethyloctylbromide was added dropwise. After complete addition the reaction mixture was heated to reflux during 16 hours. The organic layer was separated, washed with water, dried over $MgSO_4$, filtered and concentrated. 9.3 g (76 %) of a product was obtained after column chromatography ($SiO_2$, hexane/dichloromethane, 80/20, v/v).
$^1$H NMR ($CDCl_3$): δ 8.24 (s, 1H), 8.00 (dd, *J*=1.5 Hz, *J*=8 Hz, 1H), 7.42 (dt, *J*=1.5 Hz, *J*=8 Hz, 1H), 7.38 (dd, *J*=1.5 Hz, *J*=8 Hz, 1H), 7.24 (dt, *J*=1.5 Hz, *J*=8 Hz, 1H), 6.88 (s, 1H), 4.27 (t, *J*=8 Hz, 2H), 4.03 (s, 3H), 1.98-1.10 (m, 10H), 1.05 (d, *J*=6.5 Hz, 3H), 0.88 (d, *J*=6.5 Hz, 6H).
$^{13}$C NMR ($CDCl_3$): 154, 140, 140, 125, 125, 122, 120, 119, 118, 109, 103, 92, 56, 41, 39, 37, 35,31,25,23,23,20.
mp: 55 ˚C.

*Synthesis example 14*

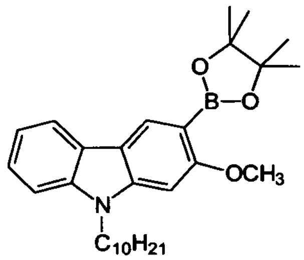

9-(3,7-dimethyloctyl)-2-methoxy-3-(4,4,5,5-tetramethyl-1,3,2-dioxaborolyl)carbazole

A solution of 5.6 g (13 mmol) 3-bromo-9-(3,7-dimethyloctyl)-2-methoxycarbazole in 75 ml tetrahydrofuran was cooled to -78˚C. 7 ml (18 mmol) 2.5 M n-butyllithium was added dropwise. After 1 hour 3.4 ml (16 mmol) 2-isopropoxy-4,4,5,5-tetramethyl-1,3,2-dioxaborolane was added dropwise. The reaction mixture was allowed to warm to room temperature overnight. The THF was evaporated and the product was purified by extraction with diethylether and water. The organic layer was dried ($MgSO_4$), filtered and concentrated. 5.9 g (95 %) of product was used without further purification.
$^1$H NMR ($CDCl_3$): δ 8.46 (s, 1H), 8.06 (dd, *J*=1.5 Hz, *J*=8 Hz, 1H), 7.38 (dt, *J*=1.5 Hz, *J*=8 Hz, 1H), 7.37 (dd, *J*=1.5 Hz, *J*=8 Hz, 1H), 7.24 (dt, *J*=1.5 Hz, *J*=8 Hz, 1H), 6.80 (s, 1H), 4.27 (t, *J*=8 Hz, 2H), 3.95 (s, 3H), 1.95-1.10 (m, 10H), 1.40 (s, 12H), 1.05 (d, *J*=6.5 Hz, 3H), 0.88 (d, *J*=6.5 Hz, 6H).
$^{13}$C NMR ($CDCl_3$): 164, 144, 140, 130, 124, 123, 120, 119, 116, 108, 91, 83, 56, 41, 39, 37, 35,31,31,28,25,24,22,22,20,14.

*Synthesis example 15*

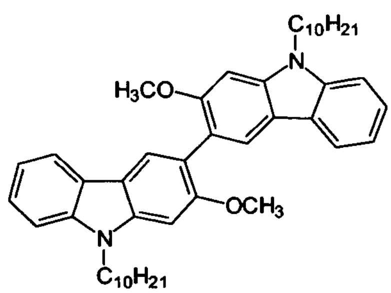

9,9'-bis(3,7-dimethyloctyl)-2,2'-dimethoxy-3,3'-bicarbazolyl

A flask, covered with aluminium foil, was charged with 2.7 g (6.5 mmol) 3-bromo-9-(3,7-dimethyl-octyl)-2-methoxycarbazole, 3.0 g (6.5 mmol) 9-(3,7-dimethyloctyl)-2-methoxy-3-(4,4,5,5-tetramethyl-1,3,2-dioxaborolyl)carbazole, 10 ml toluene and 10 ml 2 M potassium carbonate (aq). The mixture was evacuated and charged with argon for three times, after which 2 mol% $Pd(PPh_3)_4$ was added. Evacuation and filling with argon was repeated once and the mixture was stirred for 20 hours at reflux temperature. The mixture was allowed to cool to room temperature, the organic layer was separated, dried ($MgSO_4$), filtered and concentrated. After column chromatography ($SiO_2$, hexane/dichloromethane, 70/30, v/v) and crystallisation (ethanol) 1.9 g (44 %) of product was obtained.
$^1$H NMR ($CDCl_3$): δ 8.05 (s, 2H), 8.03 (dd, *J*=1.5 Hz, *J*=8 Hz, 2H), 7.43 (dt, *J*=1.5 Hz, *J*=8 Hz, 2H), 7.42 (dd, *J*=1.5 Hz, *J*=8 Hz, 2H), 7.23 (dt, *J*=1.5 Hz, *J*=8 Hz, 2H), 6.95 (s, 2H), 4.37 (t, *J*=8 Hz, 4H), 3.95 (s, 6H), 2.03-1.15 (m, 20H), 1.07 (d, *J*=6.5 Hz, 6H), 0.88 (d, *J*=6.5 Hz, 12H).
$^{13}$C NMR ($CDCl_3$): δ 157, 141, 140, 124, 123, 121, 120, 119, 116, 108, 91, 56, 41, 39, 37, 36, 31, 28, 25, 23, 23, 20.
mp: 139 ˚C.

*Synthesis example 16*

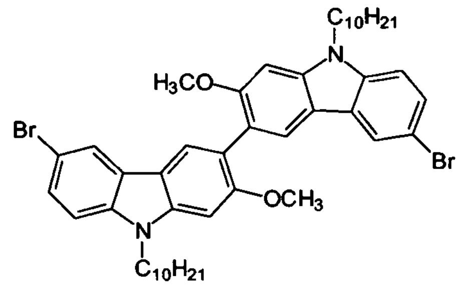

6,6'-dibromo-9,9'-bis(3,7-dimethyloctyl)-2,2'-dimethoxy-3,3'-bicarbazolyl

In a flask, covered with aluminum foil, a stirred solution of 0.5 g (0.74 mmol) 9,9'-bis(3,7-dimethyloctyl)-2,2'-dimethoxy)-3,3'-bicarbazolyl in 5 ml tetrahydrofuran was cooled to 0 ˚C. 0.25 g (1.4 mmol) N-bromosuccinimide was added in small portions. The mixture was allowed to warm to room temperature overnight. THF was evaporated. After extraction with dichloromethane and saturated $Na_2CO_3$ (aq) 0.57 g (98%) of product was obtained. This was used without further purification.
$^1$H NMR ($CDCl_3$): δ 8.10 (d, *J*=1.5 Hz, 2H), 7.96 (s, 2H), 7.49 (dd, *J*=1.5 Hz, *J*=8 Hz, 2H), 7.25 (d, *J*=8 Hz, 2H), 6.94 (s, 2H), 4.30 (t, *J*=8 Hz, 4H), 3.95 (s, 6H), 2.00-1.15 (m, 20H), 1.10 (d, *J*=6.5 Hz, 6H), 0.88 (d, *J*=6.5 Hz, 12H).
$^{13}$C NMR ($CDCl_3$): δ 158, 141, 139, 127, 125, 123, 122, 121, 115, 112, 110, 91, 56, 41, 39, 37, 35, 31, 28, 25, 23, 23, 20.

*Synthesis example 17*

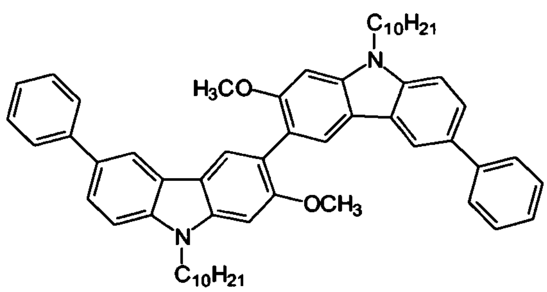

9,9'-bis(3,7-dimethyloctyl)-2,2'-dimethoxy-6,6'-diphenyl-3,3'-bicarbazolyl

A flask, covered with aluminum foil, was charged with 0.2 g (0.24 mmol) 6,6'-dibromo-9,9'-bis(3,7-dimethyloctyl)-2,2'-dimethoxy-3,3'-bicarbazolyl, 0.13 g (0.6 mmol) 4,4,5,5-tetramethyl-1,3,2-dioxaborolylbenzene, 5 ml toluene and 5 ml 2 M potassium carbonate (aq). The mixture was evacuated and charged with argon for three times, after which 2 mol% $Pd(PPh_3)_4$ was added. Evacuation and filling with argon was repeated once and the mixture was stirred for 24 hours at 105 ˚C. The mixture was allowed to cool to room temperature, the organic layer was separated, dried ($MgSO_4$), filtered and concentrated. After column chromatography ($SiO_2$, hexane/dichloromethane, 60/40, v/v) 0.07 g (35 %) of product was obtained.
$^1$H NMR ($CDCl_3$): δ8.28 (d, *J*=1.5 Hz, 2H), 8.14 (s, 2H), 7.75 (dd, *J*=1.5 Hz, *J*=8 Hz, 4H), 7.71 (dd, *J*=1.5 Hz, *J*=8 Hz, 2H), 7.50 (t, *J*=8 Hz, 4H), 7.48 (d, *J*=8 Hz, 2H), 7.36 (dt, *J*=1.5 Hz, *J*=8 Hz, 2H), 6.95 (s, 2H), 4.40 (t, *J*=8 Hz, 4H), 3.95 (s, 6H), 2.05-1.15 (m, 20H), 1.10 (d, *J*=6.5 Hz, 6H), 0.88 (d, *J*=6.5 Hz, 12H).

$^{13}C$ NMR ($CDCl_3$): δ 157, 142, 141, 140, 132, 129, 128, 126, 124, 124, 121, 118, 116, 108, 91,56,41,39,37,36,31,28,25,23,23,20.
mp: 155 ˚C.

*Synthesis example 18*

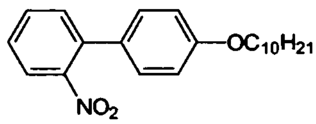

4'-(3,7-dimethyloctyloxy)-2-nitro-1,1'-biphenyl

A flask, covered with aluminum foil, was charged with 11.2 g (55 mmol) 1-bromo-2-nitrobenzene, 23.9 g (66 mmol) 1-(3,7-dimethyloctyloxy)-4-(4,4,5,5-tetramethyl-1,3,2-dioxaborolyl)benzene, 60 ml toluene and 60 ml 2 M potassium carbonate (aq). The mixture was evacuated and charged with argon for three times, after which 2 mol% $Pd(PPh_3)_4$ was added. Evacuation and filling with argon was repeated once and the mixture was stirred for 60 hours at reflux temperature. The mixture was allowed to cool to room temperature and water was added. The organic layer was separated, dried ($MgSO_4$), filtered and concentrated. After column chromatography ($SiO_2$, hexane/dichloromethane, 80/20, v/v) 12.9 g (66 %) of product was obtained.
$^1H$ NMR ($CDCl_3$): δ 7.80 (dd, *J*=1.5 Hz, *J*=8 Hz, 1H), 7.58 (dt, *J*=1.5 Hz, *J*=8 Hz, 1H), 7.44 (dd, *J*=1.5 Hz, *J*=8 Hz, 1H), 7.43 (dt, *J*=1.5 Hz, *J*=8 Hz, 1H), 7.25 (d, *J*=8 Hz, 2H), 6.96 (d, *J*=8 Hz, 2H), 4.02 (t, *J*=8 Hz, 2H), 1.90-1.13 (m, 10H), 0.97 (d, *J*=6.5 Hz, 3H), 0.88 (d, *J*=6.5 Hz, 6H),
$^{13}C$ NMR ($CDCl_3$): δ 159, 149, 136, 132, 132, 129, 129, 128, 124, 115, 66, 39, 37, 36, 30, 28, 25, 23, 23, 20.

*Synthesis example 19*

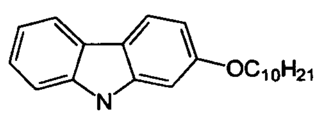

2-(3,7-dimethyloctyloxy)carbazole

13 g (36.6 mmol) 4'-(3,7-dimethyloctyloxy)-2-nitro-1,1'-biphenyl in 33 ml triethylphosphite is refluxed during 16 hours in argon atmosphere. The mixture was allowed to cool to room temperature. After evaporation of the triethylphosphite the product solidified. Filtration and washing with methanol yielded 10.5 g (89 %) of product as a white solid.
$^1H$ NMR (DMSO-$d_6$): δ 11.0 (s, 1H), 7.95 (d, *J*=8 Hz, 1H), 7.92 (d, *J*=8 Hz, 1H), 7.39 (d, J=8 Hz, 1H), 7.25 (t, *J*=8 Hz, 1H), 7.17 (t, *J*=8 Hz, 1H), 6.93 (d, *J*=1.5 Hz, 1H), 6.73 (dd, *J*=1.5 Hz, *J*=8 Hz, 1H), 4.02 (t, *J*=8 Hz, 2H), 1.80-1.10 (m, 10H), 0.92 (d, *J*=6.5 Hz, 3H), 0.82 (d, *J*=6.5 Hz, 6H).
$^{13}C$ NMR (DMSO-$d_6$): δ 158, 141, 140, 124, 123, 121, 119, 118, 116, 111, 108, 95, 66, 39, 37, 36, 29, 27, 24, 23, 23, 20.
mp: 188 ˚C.

*Synthesis example 20*

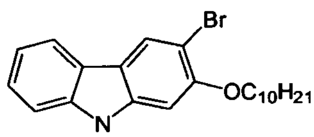

3-bromo-2-(3,7-dimethyloctyloxy)carbazole

In a flask, covered with aluminum foil, a stirred solution of 10.5 g (32.5 mmol) 2-(3,7-dimethyloctyloxy)carbazole in 40 ml tetrahydrofuran was cooled to 0 ˚C. 5.20 g (29.2 mmol) N-bromosuccinimide was added is small portions. The mixture was allowed to warm to room temperature overnight. THF was evaporated and the product was used without further purification.
$^{1}H$ NMR (DMSO-$d_6$): δ 11.20 (s, 1H), 8.33 (s, 1H), 8.05 (dd, *J*=1.5 Hz, *J*=8 Hz, 1H), 7.46 (dd, *J*=1.5 Hz, *J*=8 Hz, 1H), 7.33 (dt, *J*=1.5 Hz, *J*=8 Hz, 1H), 7.14 (dt, *J*=1.5 Hz, *J*=8 Hz, 1H), 7.14 (s, 1H), 4.15 (t, *J*=8 Hz, 2H), 1.95-1.10 (m, 10H), 0.98 (d, *J*=6.5 Hz, 3H), 0.83 (d, *J*=6.5 Hz, 6H).
$^{13}C$ NMR (DMSO-$d_6$): 153, 140, 140, 125, 122, 120, 119, 117, 111, 111, 102, 96, 67, 39, 37, 36, 29, 27, 24, 23, 23, 20.

*Synthesis example 21*

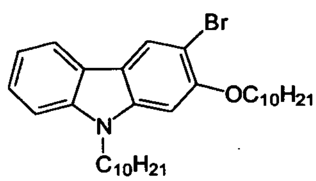

3-bromo-9-(3,7-dimethyloctyl)-2-(3,7-dimethyloctyloxy)carbazole

To a stirred solution of 13 g (32.3 mmol) 3-bromo-2-(3,7-dimethyloctyloxy)carbazole and 0.2 g benzyltriethylammoniumchloride in 35 ml toluene was added dropwise 20 g 50 w% NaOH (aq). Afterwards 8.6 g (38.9 mmol) 3,7-dimethyloctylbromide was added dropwise. After complete addition the reaction mixture was heated to reflux during 60 hours. The organic layer was separated, washed with water, dried over $MgSO_4$, filtered and concentrated. 10.1 g (57 %) of a pale yellow oil was obtained after column chromatography ($SiO_2$, hexane/dichloromethane, 80/20, v/v).
$^{1}H$ NMR ($CDCl_3$): δ8.23 (s, 1H), 7.99 (dd, *J*=1.5 Hz, *J*=8 Hz, 1H), 7.41 (dt, *J*=1.5 Hz, *J*=8 Hz, 1H), 7.38 (dd, *J*=1.5 Hz, *J*=8 Hz, 1H), 7.23 (dt, *J*=1.5 Hz, *J*=8 Hz, 1H), 6.87 (s, 1H), 4.30-4.12 (m, 4H), 2.07-1.10 (m, 20H), 1.07 (d, *J*=6.5 Hz, 3H), 1.03 (d, *J*=6.5 Hz, 3H), 0.92 (d, *J*=6.5 Hz, 6H), 0.88 (d, *J*=6.5 Hz, 6H).
$^{13}C$ NMR ($CDCl_3$): δ 154, 140, 140, 125, 124, 122, 120, 119, 117, 108, 103, 93, 67, 41, 39, 37, 37, 36, 35, 31, 30, 28, 28, 26, 25, 25, 23, 23, 20.

*Synthesis example 22*

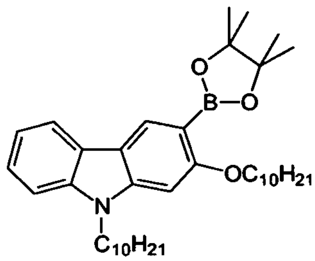

9-(3,7-dimethyloctyl)-2-(3,7-dimethyloctyloxy)-3-(4,4,5,5-tetramethyl-1,3,2-dioxaborolyl) carbazole

A solution of 4.81 g (9 mmol) 3-bromo-9-(3,7-dimethyloctyl)-2-(3,7-dimethyloctyloxy)carbazole in 40 ml tetrahydrofuran was cooled to - 78 ˚C. 4.6 ml (11.5 mmol) 2.5 M n-butyllithium was added dropwise. After 1 hour 2.2 ml (10.8 mmol) 2-isopropoxy-4,4,5,5-tetramethyl-1,3,2-dioxaborolane was added dropwise. The reaction mixture was allowed to warm to room temperature overnight.
The THF was evaporated and the product was purified by extraction with diethylether and water. The organic layer was dried ($MgSO_4$), filtered and concentrated. 3.7 g (70 %) product was used without further purification.
$^1$H NMR ($CDCl_3$): δ 8.42 (s, 1H), 8.06 (dd, *J*=1.5 Hz, *J*=8 Hz, 1H), 7.38 (dt, *J*=1.5 Hz, *J*=8 Hz, 1H), 7.37 (dd, *J*=1.5 Hz, *J*=8 Hz, 1H), 7.24 (dt, *J*=1.5 Hz, *J*=8 Hz, 1H), 6.80 (s, 1H), 4.28 (t, *J*=8 Hz, 2H), 4.18 (t, *J*=8 Hz, 2H), 1.98-1.10 (m, 20H), 1.05 (d, *J*=6.5 Hz, 3H), 1.01 (d, *J*=6.5 Hz, 3H), 0.98 (d, *J*=6.5 Hz, 6H), 0.97 (d, *J*=6.5 Hz, 6H).
$^{13}$C NMR ($CDCl_3$): δ 163, 144, 140, 129, 124, 123, 120, 119, 116, 108, 92, 83, 67, 41, 39, 37, 37, 36, 35, 31, 30, 28, 28, 26, 25, 25, 23, 23, 20.

*Synthesis example 23*

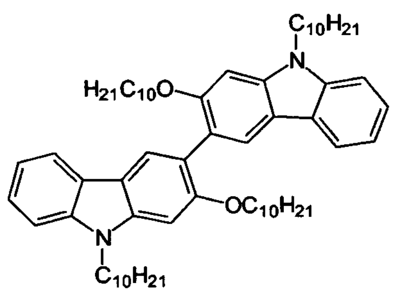

9,9'-bis(3,7-dimethyloctyl)-2,2'-bis(3,7-dimethyloctyloxy)-3,3'-bicarbazolyl

A flask, covered with aluminum foil, was charged with 0.5 g (0.9 mmol) 3-bromo-9-(3,7-dimethyloctyl)-2-(3,7-dimethyloctyloxy)carbazole, 0.65 g (1.1 mmol) 9-(3,7-dimethyloctyl)-2-(3,7-dimethyloctyloxy)-3-(4,4,5,5-tetramethyl-1,3,2-dioxaborolyl)carbazole, 5 ml toluene and 5 ml 2 M potassium carbonate (aq). The mixture was evacuated and charged with argon for three times, after which 2 mol% $Pd(PPh_3)_4$ was added. Evacuation and filling with argon was repeated once and the mixture was stirred for 48 hours at 105 ˚C. The mixture was allowed to cool to room temperature, the organic layer was separated, dried ($MgSO_4$), filtered and concentrated. After column chromatography ($SiO_2$, hexane/dichloromethane, 80/20, v/v) 0.55 g (65 %) of product was obtained.
$^1$H NMR ($CDCl_3$): δ 8.03 (s, 2H), 7.97 (dd, *J*=1.5 Hz, *J*=8 Hz, 2H), 7.41-7.36 (m, 4H), 7.21-7.14 (m, 2H), 6.91 (s, 2H), 4.30 (t, *J*=8 Hz, 4H), 4.07 (t, *J*=8 Hz, 4H), 1.98-1.00 (m, 40H), 1.10 (d, *J*=6.5 Hz, 6H), 0.85 (d, *J*=6.5 Hz, 12H), 0.80 (d, *J*=6.5 Hz, 6H), 0.78 (d, *J*=6.5 Hz, 12H).
$^{13}$C NMR ($CDCl_3$) δ 157, 141, 140, 124, 123, 122, 119, 119, 116, 108, 92, 67, 41, 39, 39, 37, 37, 36, 36, 31, 30, 28, 28, 25, 23, 23, 23, 20, 20.

*Synthesis example 24*

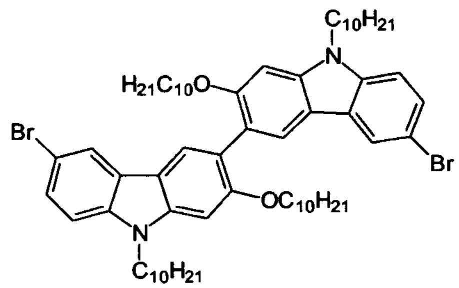

6,6'-dibromo-9,9'-bis(3,7-dimethyloctyl)-2,2'-bis(3,7-dimethyloctyloxy)-3,3'-bicarbazolyl

In a flask, covered with aluminum foil, a stirred solution of 0.42 g (0.45 mmol) 9,9'-bis(3,7-dimethyloctyl)-2,2'-bis(3,7-dimethyloctyloxy)-3,3'-bicarbazolyl in 5 ml tetrahydrofuran was cooled to 0 ˚C. 0.15 g (0.84 mmol) N-bromosuccinimide was added in small portions. The mixture was allowed to warm to room temperature overnight. THF was evaporated. After extraction with dichloromethane and saturated $Na_2CO_3$ (aq) 0.37 g (75%) of product was obtained.
$^1$H NMR ($CDCl_3$): δ 8.07 (d, *J*=1.5 Hz, 2H), 7.98 (s, 2H), 7.48 (dd, *J*=1.5 Hz, *J*=8 Hz, 2H), 7.24 (d, *J*=8 Hz, 2H), 6.91 (s, 2H), 4.30 (t, *J*=8 Hz, 4H), 4.08 (t, *J*=8 Hz, 4H), 1.98-1.00 (m, 40H), 1.10 (d, *J*=6.5 Hz, 12H), 0.88 (d, *J*=6.5 Hz, 12H), 0.78 (d, *J*=6.5 Hz, 12H).
$^{13}$C NMR ($CDCl_3$): δ 157, 141, 139, 127, 125, 124, 122, 122, 115, 112, 110, 92, 67, 41, 39, 39, 37, 37, 36, 35, 31, 30, 28, 28, 25, 23, 23, 23, 20, 20.

*Synthesis example 25*

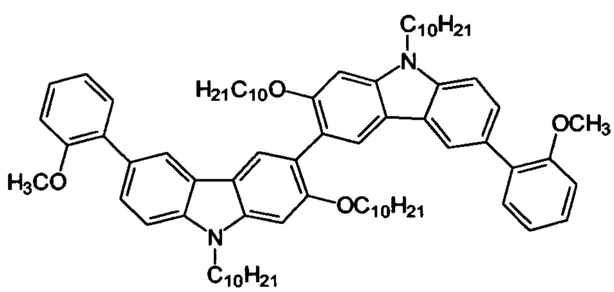

9,9'-bis(3,7-dimethyloctyl)-2,2'-bis(3,7-dimethyloctyloxy)-6,6'-bis(2-methoxyphenyl)-3,3'-bicarbazolyl

A flask, covered with aluminum foil, was charged with 2.10 g (1.9 mmol) 6,6'-dibromo-9,9'-bis(3,7-dimethyloctyl)-2,2'-bis(3,7-dimethyloctyloxy)-3,3'-bicarbazolyl, 1.3 g (5.5 mmol) 2-(4,4,5,5-tetramethyl-1,3,2-dioxaborolyl)anisole, 10 ml of toluene and 10 ml 2 M potassium carbonate (aq). The mixture was evacuated and charged with argon for three times, after which 2 mol% $Pd(PPh_3)_4$ was added. Evacuation and filling with argon was repeated once and the mixture was stirred for 60 hours at 105 ˚C. The mixture was allowed to cool to room temperature, the organic layer was separated, dried ($MgSO_4$), filtered and concentrated. After column chromatography ($SiO_2$, hexane/dichloromethane, 60/40, v/v) 0.42 g (21 %) of product was obtained.
$^1$H NMR ($CDCl_3$): δ 8.17 (d, *J*=1.5 Hz, 2H), 8.07 (s, 2H), 7.61 (dd, *J*=1.5 Hz, *J*=8 Hz, 2H), 7.47 (dd, *J*=1.5 Hz, *J*=8 Hz, 2H), 7.43 (d, *J*=8 Hz, 2H), 7.33 (dt, *J*=1.5 Hz, *J*=8 Hz, 2H), 7.08 (t, *J*=8 Hz, 2H), 7.04 (d, *J*=8 Hz, 2H), 6.96 (s, 2H), 4.35 (t, *J*=8 Hz, 4H), 4.08 (t, *J*=8 Hz, 4H), 3.83 (s, 6H), 2.03-1.02 (m, 40H), 1.10 (d, *J*=6.5 Hz, 6H), 0.93 (d, *J*=6.5 Hz, 12H),

0.85 (d, *J*=6.5 Hz, 6H), 0.80 (d, *J*=6.5 Hz, 12H).
$^{13}C$ NMR ($CDCl_3$): δ 157, 157, 149, 141, 139, 132, 131, 129, 128, 126, 123, 122, 121, 121, 116, 111, 108, 92, 67, 56, 41, 39, 39, 37, 37, 36, 36, 31, 30, 28, 28, 25, 25, 23, 23, 23, 20, 20.

*Synthesis example 26*

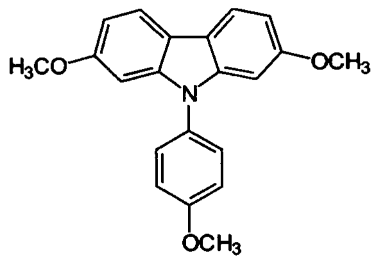

2,7-dimethoxy-9-(4-methoxyphenyl)carbazole

A mixture of 0.5 g (2 mmol) 2,7-dimethoxycarbazole, 1.0 g (4 mmol) 4-iodoanisole, 0.35 g (2.5 mmol) potassium carbonate, 0.15 g (2 mmol) copper and 23 mg 18-crown-6 in 5 ml 1,2-dichlorobenzene was evacuated, charged with argon and refluxed during 20 hours. Afterwards, the reaction mixture was allowed to cool to room temperature and filtered. Then an extraction of the filtrate using toluene and water was performed, followed by drying the organic layer over $MgSO_4$, filtration and evaporation of the solvents. 0.64 g (86 %) of product was obtained after column chromatography ($SiO_2$, gradient from pure hexane to pure dichloromethane) followed by crystallisation from dichloromethane and methanol.
$^{1}H$ NMR ($CDCl_3$): δ 7.90 (d, *J*=8 Hz, 2H), 7.44 (d, *J*=8 Hz, 2H), 7.12 (d, *J*=8 Hz, 1H), 6.86 (dd, *J*=1.5 Hz, *J*=8 Hz, 2H), 6.75 (d, *J*=1.5 Hz, 2H), 3.92 (s, 3H), 3.82 (s, 6H).
$^{13}C$ NMR ($CDCl_3$): δ 159, 158, 143, 130, 129, 120, 117, 115, 108, 94, 56, 56.
mp: 143 ˚C.

*Synthesis example 27*

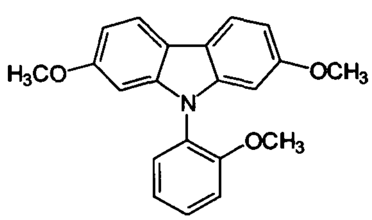

2,7-dimethoxy-9-(2-methoxyphenyl)carbazole

A mixture of 0.5 g (2 mmol) 2,7-dimethoxycarbazole, 1.0 g (4 mmol) 2-iodoanisole, 0.35 g (2.5 mmol) potassium carbonate, 0.15 g (2 mmol) copper and 23 mg 18-crown-6 in 5 ml 1,2-dichlorobenzene was evacuated, charged with argon and refluxed during 40 hours. Afterwards, the reaction mixture was allowed to cool to room temperature and filtered. Then an extraction of the filtrate using toluene and water was performed, followed by drying the organic layer over $MgSO_4$, filtration and evaporation of the solvents. 0.6 g (81 %) of product was obtained after column chromatography ($SiO_2$, gradient from pure hexane to pure dichloromethane).
$^{1}H$ NMR ($CDCl_3$): δ 7.90 (d, *J*=8 Hz, 2H), 7.50 (dt, *J*=1.5 Hz, *J*=8 Hz, 1H), 7.45 (dd, *J*=1.5 Hz, *J*=8 Hz, 1H), 7.18 (dd, *J*=1.5 Hz, *J*=8 Hz, 1H), 7.16 (dt, *J*=1.5 Hz, *J*=8 Hz, 1H), 6.86 (dd, *J*=1.5 Hz, *J*=8 Hz, 2H), 6.58 (d, *J*=1.5 Hz, 2H), 3.82 (s, 6H), 3.73 (s, 3H).

$^{13}C$ NMR ($CDCl_3$): δ 158, 156, 143, 130, 130, 126, 121, 120, 117, 113, 108, 95, 56.
mp: 148°C.

*Synthesis example 28*

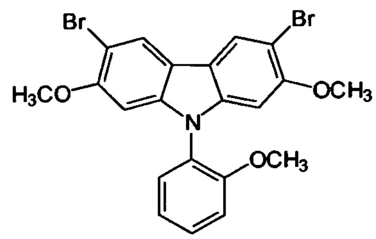

3,6-dibromo-2,7-dimethoxy-9-(2-methoxyphenyl)carbazole

In a flask, covered with aluminum foil, a stirred solution of 0.17 g (0.5 mmol) 2,7-dimethoxy-9-(2-methoxyphenyl) carbazole in 5 ml tetrahydrofuran was cooled to 0 °C. 0.25 g (1.4 mmol) N-bromosuccinimide was added in small portions. The mixture was allowed to warm to room temperature overnight. THF was evaporated. After extraction with dichloromethane and saturated $Na_2CO_3$ (aq) 0.25 g (99%) of product was obtained as a white solid after column chromatography ($SiO_2$, dichloromethane).
$^1H$ NMR ($CDCl_3$): δ 8.11 (s, 2H), 7.55 (dt, *J*=1.5 Hz, *J*=8 Hz, 1H), 7.43 (dd, *J*=1.5 Hz, *J*=8 Hz, 1H), 7.22-7.17 (m, 2H), 6.86 (dd, *J*=1.5 Hz, *J*=8 Hz, 2H), 6.54 (s, 2H), 3.82 (s, 6H), 3.73 (s, 3H).
$^{13}C$ NMR ($CDCl_3$): δ 156, 154, 142, 130, 130, 125, 124, 121, 117, 113, 104, 94, 57, 56.

While the invention has been described in detail and with reference to specific embodiments thereof, it will be apparent for one skilled in the art that various changes and modifications can be made therein without departing from the spirit and scope thereof.

## Claims

**1.** A carbazole compound having the general formula (I)

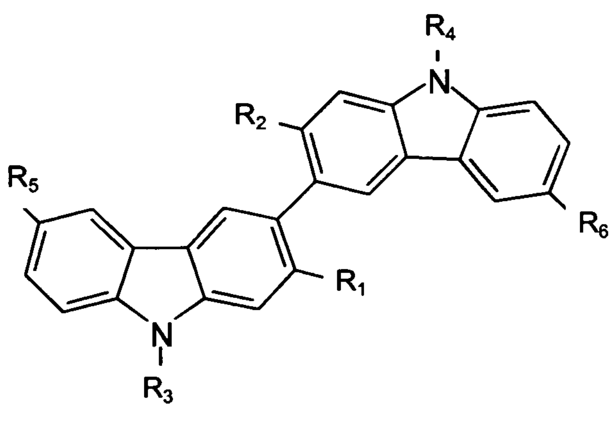

(I)

wherein

-$R_1$, and -$R_2$ are, the same or different at each occurrence, -$OR^{41}$, -$OR^{42}$, -$SR^{41}$, -$SR^{42}$, -$NR^{41}R^{45}$, or -$NR^{42}R^{45}$;
-$R_3$, and -$R_4$ are, the same or different at each occurrence, $R^{41}$, or $R^{42}$, with
$R^{41}$ being $C_1$-$C_{20}$ cyclic or acyclic straight or branched alkyl optionally interrupted one or more times with -O-, -OC(=O)-, -C(=O)O-, -S-, secondary nitrogen, tertiary nitrogen, quaternary nitrogen, -$CR^{45}$=$CR^{46}$-, -C≡C-, -C(=O)-, -C(=O)$NR^{45}$-, - $NR^{45}$C(=O)-, -S(=O)-, $-S(=O)_2-$, or $-X^6-$, and/or substituted one or more times with $R^{42}$, $R^7$, or $R^8$;
$R^{42}$ being $C_5$-$C_{30}$ aryl in which, optionally, one or more of the aromatic carbon atoms are replaced with N, O or S, and, optionally, one or more of the aromatic carbon atoms carry a group $R^{41}$, $R^7$, or $R^8$;
$R^7$ being -CN, $-CF_3$, -CSN, $-NH_2$, $-NO_2$, -NCO, -NCS, -OH, -F, $-PO_2$, $-PH_2$, -SH, -Cl, -Br, or -I;
$R^8$ being $-C(=O)R^{45}$, $-C(=O)OR^{45}$, $-C(=O)NR^{45}R^{46}$, $-NHR^{45}$,$-NR^{45}R^{46}$, $-N^{(+)}R^{45}R^{46}R^{47}$, $-NC(=O)R^{45}$, $-OR^{45}$, $-OC(=O)R^{45}$, $-SR^{45}$, $-S(=O)R^{45}$, or - $S(=O)_2R^{45}$;
$R^{45}$, $R^{46}$, and $R^{47}$ being, the same or different at each occurrence, H, $R^{41}$, or $R^{42}$;
$X^6$ being $C_4$-$C_{30}$ arylene in which, optionally, one or more of the aromatic carbon atoms are replaced with N, 0, or S, and, optionally, one or more of the aromatic carbon atoms carry a group $R^{41}$, $R^7$, or $R^8$; and
$-R_5$, and $-R_6$ are, the same or different at each occurrence, H, $R^{7'}$, $R^{41'}$, or $R^{42'}$, with
$R^{41'}$ being $C_1$-$C_{20}$ cyclic or acyclic straight or branched alkyl, optionally interrupted one or more times with -O-, -OC(=O)-, -C(=O)O-, -S-, secondary nitrogen, tertiary nitrogen, quaternary nitrogen, $-CR^{45'}=CR^{46'}-$, -C≡C-, -C (=O)-, - C(=O)$NR^{45'}$-, - $NR^{45'}$C(=O)-, -S(=O)-, $-S(=O)_2-$, or $-X^{6'}-$; and/or substituted one or more times with $R^{42'}$, $R^{7'}$, or $R^{8'}$;
$R^{42'}$ being $C_5$-$C_{30}$ aryl, in which one or more of the aromatic carbon atoms in ortho position carry a group $R^{41'}$, $R^{45'}$, $R^{7'}$, or $R^{8'}$, and, optionally, one or more of the aromatic carbon atoms are replaced with N, O or S, and, optionally, one or more of the aromatic carbon atoms carry a group $R^{41'}$, $R^{7'}$, or $R^{8'}$;
$R^{7'}$ being -CN, $-CF_3$, -CSN, $-NH_2$, $-NO_2$, -NCO, -NCS, -OH, -F,$-PO_2$, $-PH_2$, -SH, -Cl, -Br, -I, or $-B(OR^{41'})(OR^{45'})$;
$R^{8'}$ being $-C(=O)R^{45'}$, $-C(=O)OR^{45'}$, $-C(=O)NR^{45'}R^{46'}$, $-NHR^{45'}$,$-NR^{45'}R^{46'}$, $-N^{(+)}R^{45'}R^{46'}R^{47'}$, $-NC(=O)R^{45'}$-, $-OR^{45'}$, $-OC(=O)R^{45'}$, $-SR^{45'}$, $-S(=O)R^{45'}$, or - $S(=O)_2R^{45'}$;
$R^{45'}$, $R^{46'}$, $R^{47'}$ being, the same or different at each occurrence, H, $R^{41'}$, or $R^{42'}$;
$X^{6'}$ being $C_4$-$C_{30}$ arylene in which, optionally, one or more of the aromatic carbon atoms are replaced with N, O, or S, and, optionally, one or more of the aromatic carbon atoms carry a group $R^{41'}$, $R^{7'}$, or $R^{8'}$.

2. A compound according to claim 1, wherein each of $R_1$ and $R_2$ is $-OR^{41}$.

3. A compound according to claim 2, wherein $-OR^{41}$ is methyloxy ($-OCH_3$).

4. A compound according to claim 2, wherein $-OR^{41}$ is straight or branched chain decyloxy ($-OC_{10}H_{21}$).

5. A compound according to any one of the preceding claims, wherein each of $R_3$ and $R_4$ is $R^{41}$.

6. A compound according to claim 5, wherein $R^{41}$ is straight or branched chain decyl ($-C_{10}H_{21}$).

7. A compound according to any one of the preceding claims, wherein each of $R_5$ and $R_6$ is H.

8. A compound according to any one of the claims 1-6, wherein each of $R_5$ and $R_6$ is $R^{42'}$.

9. A compound according to claim 8, wherein $R^{42'}$ is ortho-methoxyphenyl.

10. A compound according to any one of the claims 1-6, wherein each of $R_5$ and $R_6$ is $R^{7'}$.

11. A compound according to claim 10, wherein $R^{7'}$ is Br.

12. A semiconducting material comprising a compound according to any one of the preceding claims.

13. An electroluminescent device comprising a semiconducting material according to claim 12.

14. An electroluminescent device according to claim 13, wherein said semiconducting material is combined with a phosphoresent emitter.

15. A process for the preparation of a compound according to any one of the claims 1-11.

16. Use of a compound according to any one of the claims 1-11 as a semiconducting material.

17. Use of a compound according to any one of the claims 1-11 as a host matrix for phosphorescent emitters.

**Patentansprüche**

**1.** Carbazolverbindung mit der allgemeinen Formel (I)

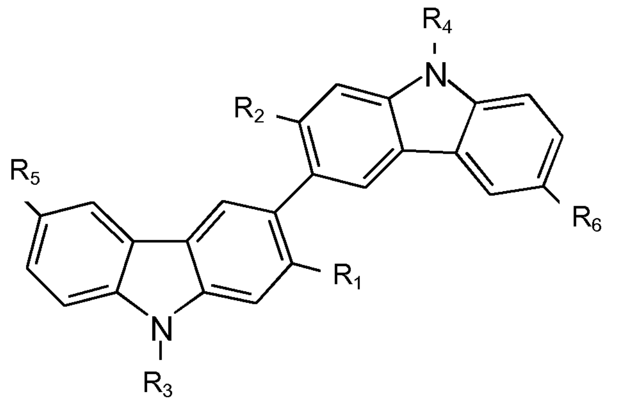

(I)

wobei

$-R_1$ und $-R_2$ gleich $-OR^{41}$, $-OR^{42}$, $-SR^{41}$, $-SR^{42}$, $-NR^{41}R^{45}$ oder $-NR^{42}R^{45}$ sind und bei jedem Auftreten gleich oder verschieden,
$-R_3$ und $-R_4$ gleich $R^{41}$ oder $R^{42}$ sind und bei jedem Auftreten gleich oder verschieden,
und
wobei $R^{41}$ zyklisches oder azyklische gerades oder verzweigtes $C_1$-$C_{20}$-Alkyl ist, optional ein oder mehrere Male durch -O-, -OC(=O)-, -C(=O)O-, -S-, sekundären Stickstoff, tertiären Stickstoff, quaternären Stickstoff, $-CR^{41}=CR^{46}-$, -C≡C-, -C(=O)-, $-C(=O)NR^{45}-$, $-NR^{45}C(=O)-$, -S(=O)-, $-S(=O)_2-$ oder $-X^6-$ unterbrochen und/oder ein oder mehrere Male durch $R^{42}$, $R^7$ oder $R^8$ substituiert;
wobei $R^{42}$ $C_5$-$C_{30}$-Aryl ist, in dem, optional, ein oder mehrere der aromatischen Kohlenstoffatome durch N, O oder S ersetzt sind und, optional, ein oder mehrere der aromatischen Kohlenstoffatome eine Gruppe $R^{41}$, $R^7$ oder $R^8$ tragen;
wobei $R^7$ gleich -CN, $-CF_3$, -CSN, $-NH_2$, $-NO_2$, -NCO, -NCS, -OH, -F, $-PO_2$, $-PH_2$, -SH, -Cl, -Br oder -I ist;
wobei $R^8$ gleich $-C(=O)R^{45}$, $-C(=O)OR^{45}$, $-C(=O)NR^{45}R^{46}$, $-NHR^{45}$, $-NR^{45}R^{46}$, $-N^{(+)}R^{45}R^{46}R^{47}$, $-NC(=O)R^{45}-$, $-OR^{45}$, $-OC(=O)R^{45}$, $-SR^{45}$, $-S(=O)R^{45}$ oder $-S(=O)_2R^{45}$ ist,
wobei $R^{45}$, $R^{46}$ und $R^{47}$ gleich H, $R^{41}$ oder $R^{42}$ sind und bei jedem Auftreten gleich oder verschieden,
wobei $X^6$ $C_4$-$C_{30}$-Arylen ist, in dem, optional, ein oder mehrere der aromatischen Kohlenstoffatome durch N, O oder S ersetzt sind und, optional, ein oder mehrere der aromatischen Kohlenstoffatome eine Gruppe $R^{41}$, $R^7$ oder $R^8$ tragen, und
$-R_5$ und $-R_6$ gleich H, $R^{7'}$, $R^{41'}$ oder $R^{42'}$ sind und bei jedem Auftreten gleich oder verschieden und
wobei $R^{41'}$ $C_1$-$C_{20}$ zyklisches oder azyklische gerades oder verzweigtes Alkyl ist, optional ein oder mehrere Male durch -O-, -OC(=O)-, -C(=O)O-, -S-, sekundären Stickstoff, tertiären Stickstoff, quaternären Stickstoff, $-CR^{45'}=CR^{46'}-$, -C≡C-, -C(=O)-, $-C(=O)NR^{45'}-$, $-NR^{45'}C(=O)-$, -S(=O)-, $-S(=O)_2-$ oder $-X^{6'}-$ unterbrochen; und/oder ein oder mehrere Male durch $R^{42'}$, $R^{7'}$ oder $R^{8'}$ substituiert;
wobei $R^{42'}$ $C_5$-$C_{30}$-Aryl ist, in dem ein oder mehrere der aromatischen Kohlenstoffatome in ortho-Position eine Gruppe $R^{41'}$, $R^{45'}$, $R^{7'}$ oder $R^{8'}$ tragen und, optional, ein oder mehrere der aromatischen Kohlenstoffatome durch N, O oder S ersetzt sind und, optional, ein oder mehrere der aromatischen Kohlenstoffatome eine Gruppe $R^{41'}$, $R^{7'}$ oder $R^{8'}$ tragen;
wobei $R^{7'}$ gleich -CN, $-CF_3$, -CSN, $-NH_2$, $-NO_2$, -NCO, -NCS, -OH, -F, $-PO_2$, $-PH_2$, -SH, -Cl, -Br, -I oder $-B(OR^{41'})(OR^{45'})$ ist;
wobei $R^{8'}$ gleich $-C(=O)R^{45'}$, $-C(=O)OR^{45'}$, $-C(=O)NR^{45'}R^{46'}$, $-NHR^{45'}$, $-NR^{45'}R^{46'}$, $-N^{(+)}R^{45'}R^{46'}R^{47'}$, $-NC(=O)R^{45'}-$, $-OR^{45'}$, $-OC(=O)R^{45'}$, $-SR^{45'}$, $-S(=O)R^{45'}$ oder $-S(=O)_2R^{45'}$ ist;
wobei $R^{45'}$, $R^{46'}$, $R^{47'}$ gleich H, $R^{41'}$ oder $R^{42'}$ sind und bei jedem Auftreten gleich oder verschieden;
wobei $X^{6'}$ $C_4$-$C_{30}$-Arylen ist, in dem, optional, ein oder mehrere der aromatischen Kohlenstoffatome durch N, O oder S ersetzt sind und, optional, ein oder mehrere der aromatischen Kohlenstoffatome eine Gruppe $R^{41'}$, $R^{7'}$ oder $R^{8'}$ tragen.

**2.** Verbindung nach Anspruch 1, bei der sowohl $R_1$ als auch $R_2$ gleich $-OR^{41}$ ist.

**3.** Verbindung nach Anspruch 2, bei der $-OR^{41}$ -Methyloxy ($-OCH_3$) ist.

**4.** Verbindung nach Anspruch 2, bei der $-OR^{41}$ gerad- oder verzweigtkettiges -Decyloxy ($-OC_{10}H_{21}$) ist.

**5.** Verbindung nach einem der vorhergehenden Ansprüche, bei der sowohl $R_3$ als auch $R_4$ gleich $R^{41}$ ist.

**6.** Verbindung nach Anspruch 5, bei der $R^{41}$ linear- oder verzweigtkettiges Decyl ($-C_{10}H_{21}$) ist.

**7.** Verbindung nach einem der vorhergehenden Ansprüche, bei der sowohl $R_5$ als auch $R_6$ gleich H ist.

**8.** Verbindung nach einem der Ansprüche 1-6, bei der sowohl $R_5$ als auch $R_6$ gleich $R^{42'}$ ist.

**9.** Verbindung nach Anspruch 8, bei der $R^{42'}$ Orthomethoxyphenyl ist.

**10.** Verbindung nach einem der Ansprüche 1-6, bei der sowohl $R_5$ als auch $R_6$ gleich $R^7$ ist.

**11.** Verbindung nach Anspruch 10, bei der $R^{7'}$ gleich Br ist.

**12.** Halbleitendes Material mit einer Verbindung nach einem der vorhergehenden Ansprüche.

**13.** Elektrolumineszenzanordnung mit einem halbleitenden Material nach Anspruch 12.

**14.** Elektrolumineszenzanordnung nach Anspruch 13, bei der das halbleitende Material mit einem Phosphoreszenzemitter kombiniert ist.

**15.** Prozess zum Zubereiten einer Verbindung nach einem der Ansprüche 1-11.

**16.** Verwendung einer Verbindung nach einem der Ansprüche 1-11 als halbleitendes Material.

**17.** Verwendung einer Verbindung nach einem der Ansprüche 1-11 als Wirtsmatrix für Phosphoreszenzemitter.

**Revendications**

**1.** Composé de carbazole de formule générale (I) :

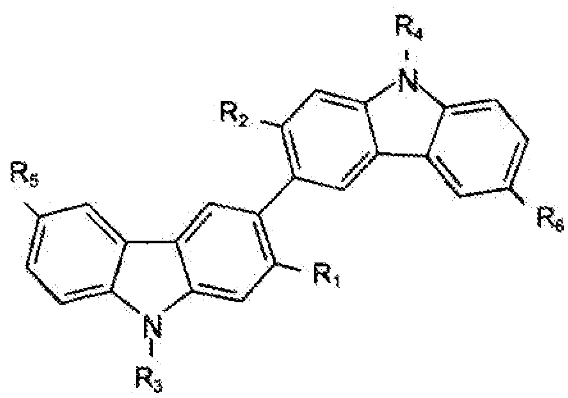

(I)

dans laquelle :

$-R_1$ et $-R_2$ sont identiques ou différents à chaque occurrence et représentent les substituants $-OR^{41}$, $-OR^{42}$ , $-SR^{41}$ , $-SR^{42}$ , $-NR^{41}R^{45}$ ou $-NR^{42}R^{45}$;
$-R_3$ et $-R_4$ sont identiques ou différents à chaque occurrence et représentent le radical $R^{41}$ ou $R^{42}$, avec :
$R^{41}$ représentant un alkyle en $C_1$-$C_{20}$ cyclique ou acyclique, linéaire ou ramifié, éventuellement interrompu une ou plusieurs fois par un élément ou un groupement du type -O-, -OC(=O)-, -C(=O)O-, -S-, azote secondaire,

azote tertiaire, azote quaternaire, $-CR^{45}=CR^{46}-$, -C≡C-, -C(=O)-, $-C(=O)NR^{45}-$, $-NR^{45}C(=O)-$, -S(=O)-, $-S(=O)_2-$ ou $-X^6-$, et/ou substitué une ou plusieurs fois par un radical $R^{42}$, $R^7$ ou $R^8$;
$R^{42}$ représentant un aryle en $C_5$-$C_{30}$ dans lequel, éventuellement, un ou plusieurs atomes de carbone aromatiques sont remplacés par un élément N, O ou S et, éventuellement, un ou plusieurs atomes de carbone aromatiques portent un groupement $R^{41}$, $R^7$ ou $R^8$;
$R^7$ représentant un substituant -CN, $-CF_3$, -CSN, $-NH_2$, $-NO_2$, -NCO, -NCS, -OH, -F, $-PO_2$, $-PH_2$, -SH, -Cl, -Br ou -I ;
$R^8$ représentant un radical $-C(=O)R^{15}$, $-C(=O)OR^{45}$, $-C(=O)NR^{45}R^{46}$, $-NHR^{45}$, $-NR^{45}R^{46}$, $-N^{(+)}R^{45}R^{46}R^{47}$, -NC $(=O)R^{45}-$, $-OR^{45}$, $-OC(=O)R^{45}$, $-SR^{45}$, $-S(=O)R^{45}$ ou $-S(=O)_2R^{45}$;
$R^{45}$, $R^{46}$ et $R^{47}$ étant identiques ou différents à chaque occurrence et représentant H, $R^{41}$ ou $R^{42}$;
$X^6$ représentant un arylène en $C_4$-$C_{30}$ dans lequel, éventuellement, un ou plusieurs atomes de carbone aromatiques sont substitués par un élément N, O ou S et, éventuellement, un ou plusieurs atomes de carbone aromatiques portent un groupement $R^{41}$, $R^7$ ou $R^8$; et
$-R_5$ et $-R_6$ sont identiques ou différents à chaque occurrence et représentent H, $R^{7'}$, $R^{41'}$, ou $R^{42'}$, avec :
$R^{41'}$ représentant un alkyle en $C_1$-$C_{20}$ cyclique ou acyclique, linéaire ou ramifié, éventuellement interrompu une ou plusieurs fois par un élément ou un groupement de type -O-, -OC(=O)-, -C(=O)O-, -S-, azote secondaire, azote tertiaire, azote quaternaire, $-CR^{45'}=CR^{46'}-$, -C≡C-, -C(=O)-, $-C(=O)NR^{45'}-$, - $NR^{45'}C(=O)-$, -S(=O)-, -S $(=O)_2-$, ou $-X^{6'}-$, et/ou substitué une ou plusieurs fois par un radical $R^{42'}$, $R^{7'}$ ou $R^{8'}$;
$R^{42'}$ représentant un aryle en $C_5$-$C_{30}$ dans lequel un ou plusieurs atomes de carbone aromatiques en position ortho portent un groupement $R^{41'}$, $R^{45'}$, $R^{7'}$ ou $R^{8'}$ et, éventuellement, un ou plusieurs atomes de carbone aromatiques sont remplacés par un élément N, O ou S et, éventuellement, un ou plusieurs des atomes de carbone aromatiques portent un groupement $R^{41'}$, $R^{7'}$ ou $R^{8'}$;
$R^{7'}$ représentant un substituant -CN, $-CF_3$, -CSN, $-NH_2$, $-NO_2$, -NCO, -NCS, -OH, -F, $-PO_2$, $-PH_2$, -SH, -Cl, -Br, -I ou $-B(OR^{41'})(OR^{45'})$;
$R^{8'}$ représentant un substituant $-C(=O)R^{45'}$, $-C(=O)OR^{45'}$, $-C(=O)NR^{45'}R^{46'}$, $-NHR^{45'}$, $-NR^{45'}R^{46'}$, $-N^{(+)}R^{45'}R^{46'}R^{47'}$, $-NC(=O)R^{45'}-$, $-OR^{45'}$, $-OC(=O)R^{45'}$, $-SR^{45'}$, $-S(=O)R^{45'}$ ou $-S(=O)_2R^{45'}$;
$R^{45'}$, $R^{46'}$, $R^{47'}$ étant identiques ou différents à chaque occurrence et représentant H, $R^{41'}$ ou $R^{42'}$;
$X^{6'}$ représentant un arylène en $C_4$-$C_{30}$ dans lequel, éventuellement, un ou plusieurs des atomes de carbone aromatiques sont substitués par un élément N, O ou S et, éventuellement, un ou plusieurs des atomes de carbone aromatiques portent un groupement $R^{41'}$, $R^{7'}$ ou $R^{8'}$.

**2.** Composé selon la revendication 1, dans lequel chacun des radicaux $R_1$ et $R_2$ est représenté par le substituant $-OR^{41}$.

**3.** Composé selon la revendication 2, dans lequel le substituant $-OR^{41}$ est le groupement méthyloxy ($-OCH_3$).

**4.** Composé selon la revendication 2, dans lequel le substituant $-OR^{41}$ est un groupement décyloxy ($-OC_{10}H_{21}$) à chaîne linéaire ou ramifiée.

**5.** Composé selon l'une quelconque des revendications précédentes, dans lequel chacun des radicaux $R_3$ et $R_4$ représente le radical $R^{41}$.

**6.** Composé selon la revendication 5, dans lequel le radical $R^{41}$ est un groupement décyle ($-C_{10}H_{21}$) à chaîne linéaire ou ramifiée.

**7.** Composé selon l'une quelconque des revendications précédentes, dans lequel chacun des radicaux $R_5$ et $R_6$ représente l'hydrogène.

**8.** Composé selon l'une quelconque des revendications 1 à 6, dans lequel chacun des radicaux $R_5$ et $R_6$ représente le radical $R^{42'}$.

**9.** Composé selon la revendication 8, dans lequel le radical $R^{42'}$ représente le groupement ortho-méthoxyphényle.

**10.** Composé selon l'une quelconque des revendications 1 à 6, dans lequel chacun des radicaux $R_5$ et $R_6$ représente le radical $R^{7'}$.

**11.** Composé selon la revendication 10, dans lequel le radical $R^{7'}$ représente le brome.

**12.** Matériau semi-conducteur, comprenant un composé selon l'une quelconque des revendications précédentes.

**13.** Dispositif électroluminescent, comprenant un matériau semi-conducteur selon la revendication 12.

**14.** Dispositif électroluminescent selon la revendication 13, dans lequel ledit matériau semi-conducteur est combiné à un émetteur phosphorescent.

**15.** Procédé pour la préparation d'un composé selon l'une quelconque des revendications 1 à 11.

**16.** Utilisation d'un composé selon l'une quelconque des revendications 1 à 11, comme matériau semi-conducteur.

**17.** Utilisation d'un composé selon l'une quelconque des revendications 1 à 11, comme matrice hôte pour des émetteurs phosphorescents.

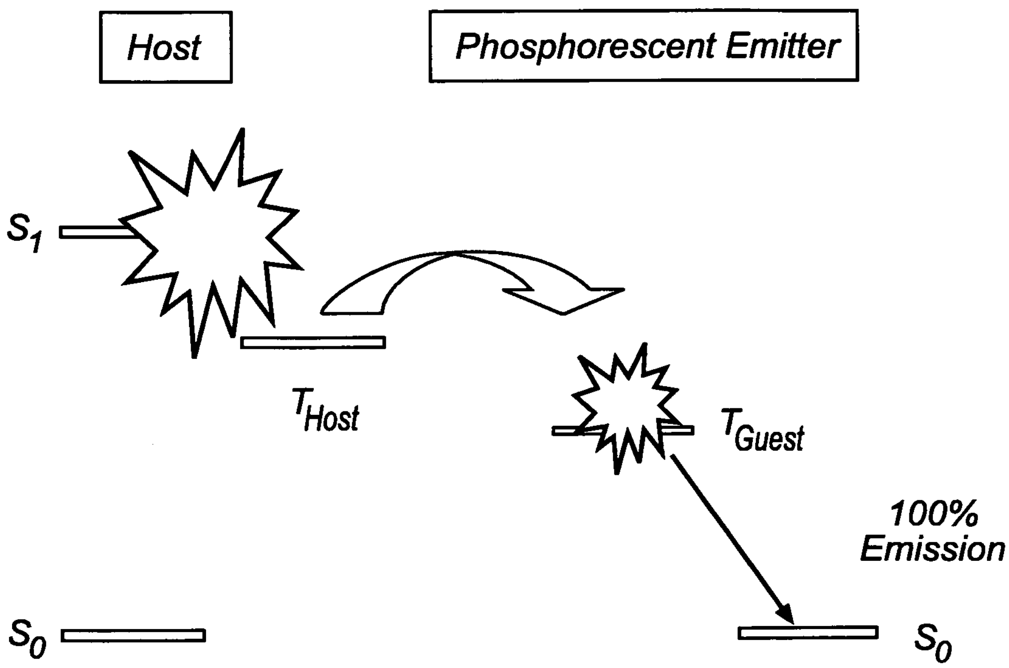
Host
Phosphorescent Emitter
$S_1$
$T_{Host}$
$T_{Guest}$
100%
Emission
$S_0$
$S_0$

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04072205 A


### Non-patent literature cited in the description


- **TOKITO, S. ; IIJIMA, T. ; SUZURI, Y. ; KITA, H. ; TSUZUKI, T. ; SATO, F.** *Appl. Phys. Lett.,* 2003, vol. 83, 569